# EUROPEAN PATENT SPECIFICATION

(11) **EP 4 184 165 B1**
(45) Date of publication and mention of the grant of the patent: **27.11.2024**
(21) Application number: 20967226.0
(22) Date of filing: 30.12.2020
(51) Int. Cl.: G01N 33/24, G01N 1/38

(54) **PORTABLE MODULAR GASTROINTESTINAL SIMULATOR SYSTEM THAT QUANTIFIES BIOACCESSIBILITY OF METALS IN A SAMPLE REMOTELY AND IN REAL TIME, AND METHOD**
TRAGBARES MODULARES GASTROINTESTINALES SIMULATORSYSTEM ZUR QUANTIFIZIERUNG DER BIOZUGÄNGLICHKEIT VON METALLEN IN EINER PROBE AUS DER FERNE UND IN ECHTZEIT SOWIE VERFAHREN
SYSTÈME MODULAIRE SIMULATEUR GASTRO-INTESTINAL, PORTATIF, QUI QUANTIFIE À DISTANCE, EN TEMPS RÉEL, LA BIOACCESSIBILITÉ DE MÉTAUX DANS UN ÉCHANTILLON, ET PROCÉDÉ

(43) Date of publication of application: 24.05.2023
(73) Proprietor: Universidad Tecnológica Metropolitana, Santiago (CL)
(72) Inventor: PARODI DÁVILA, María Carolina, 390 Santiago (CL); MIRANDA SANDOVAL, Michael, 390 Santiago (CL); ALENCAR DA SILVA, Keyla, 390 Santiago (CL)
(74) Representative: Pons
(86) International application number: PCT/CL2020/050198
(87) International publication number: WO 2022/140868

(56) References cited:
- CN-A- 1 719 250
- CN-A- 102 399 692
- CN-A- 103 439 476
- CN-A- 103 439 476
- CN-A- 103 837 655
- CN-A- 104 851 346
- CN-A- 107 978 368
- CN-A- 108 364 553
- CN-A- 109 975 524
- ES-A1- 2 361 983
- KR-B1- 101 952 612
- ROSENDE MARÍA ET AL: "Assessing oral bioaccessibility of trace elements in soils under worst-case scenarios by automated in-line dynamic extraction as a front end to inductively coupled plasma atomic emission spectrometry", ANALYTICA CHIMICA ACTA, ELSEVIER, AMSTERDAM, NL, vol. 842, 23 June 2014 (2014-06-23), pages 1 - 10, XP029045045, ISSN: 0003-2670, DOI: 10.1016/J.ACA.2014.06.033
- RIVAS-MONTOYA ERNESTO ET AL: "Application of a novel gastrointestinal tract simulator system based on a membrane bioreactor (SimuGIT) to study the stomach tolerance and effective delivery enhancement of nanoencapsulated macelignan", CHEMICAL ENGINEERING SCIENCE, OXFORD, GB, vol. 140, 17 October 2015 (2015-10-17), pages 104 - 113, XP029328977, ISSN: 0009-2509, DOI: 10.1016/J.CES.2015.10.006

## Description

### Field of the Invention

The present invention is related to a light and portable modular gastrointestinal simulator system, and a method for remotely quantifying, in real time, the concentration or quantity of metals and metalloids present in a sample of contaminated soil, preferably, mining tailings soil, which could be accessible to human body under regular conditions of human digestion (bioaccesibility) and determining risks on the human health, where such metal and metalloids are selected from the group consisting on: iron (Fe), lead (Pb), Zinc (Zn) and copper (Cu), nickel (Ni); arsenic (As) and cadmium (Cd), comprising: an upper pre-mixing module or pre-gastric preparation, an intermediate mixing or gastrointestinal or gastric simulation module, and a recirculation or lower module. The first two modules of the present system have pH, temperature and stirring rate sensors transmitting an information to a database center wherein this information is stored together preset pH, temperature and stirring rate values and the incoming information is compared to that stored, and a output signal or response is generated allowing at least one or more controlling means of pH, temperature, rate are manually or automatically to regulate pH and temperature inside the sampling vessels and stirring rate of the stirring means to achieve preset values to achieve an homogeneity percent of the mixture over 50%, preferably, 90%. Optionally, the present modular system comprising means emitting visual signals, sounding signals or both, warning to the user that time has elapsed up to achieve homogeneity of mixture over 50%, preferably 90%, in the intermediate module when sample is mixed with S and G or sample is mixed with S, G, B and D. Being enough time to achieve a 50% homogeneity in the intermediate module of half hour. When a soil sample is mixed with S and G while to achieve a 90% homogeneity in the same module when a soil sample is mixed with S and G, time is approx. 1 hour. Being enough time to achieve a 90% homogeneity in the intermediate module when a soil sample is mixed with S, G, B and D of two hours while up to achieving a 90% homogeneity in the same module when a soil sample is mixed with S, G, B and D, time is approx., is 4 hours. Bioreactor design of the gastrointestinal or gastric simulator module (intermediate module), included a stirring means, are an own design.

### Background

Currently there is an increasing necessity in the environmental or mining industry to monitoring variables to support strategic decisions to protect environment or human health, with low-cost methods including a reliable, in real time and on line releasing of results to the faithful performance of regulations in force.

One of the variables which has become more and more important the last years, is bioaccesibility since the same allowing the quantification, at a low cost, of contaminants present in a soil matrix available to be absorbed by human body and thus protecting human health of individuals exposed to the same. However, this variable can be quantified by several methods, between the most remarkable are the physiological ones: PBET (Physiologically Based Extraction Test) and UBM (Unified BARGE Methodology, Barge) to avoid animal or human tests, considering the design which is very valuated by the animal and scientific community, and that currently are implemented at level laboratory in countries such as Canada, Spain, USA e England, to perform several soil studies.

Method based on physiology using synthetic fluids substituting human bowel and gastric fluids, further variables such as temperature, pH, solid and liquid phase ratio, time and movement of digestive process. Representing fluids of several steps of the digestive process are sequentially applied simulating steps of the gastrointestinal tract.

UBM method as opposed to PBET ("Physiologically Based Extraction Test") method is performed following a procedure as described in the international norm ISO 17924 which implies that its results are comparable to the results obtained by other laboratories following the same norm. To the application of UBM a method ("Unified BARGE Methodology"), the same is validated by the analysis of the reference material BGS 102 certifying the bioaccesible metal and metalloid content in gastrointestinal and gastric phase of a digestive process. At international level Canada and England due to having laboratories like as "EnviroLabs" laboratories, located at Manchester, "Derwentside Environmental Testing Services Limited, DET"S, Laboratory located at Consett; Chemtest Ltd (https://www.chemtest.com), laboratory located at Cambridge, applying the UBM method following ISO 17.924 norm allowing that the obtained results by this method at national level are traceable at international level. In this context, British Geological Survey (https://www.bgs.ac.uk/) stands out, who is a worldwide organization leading global geological and geoscience studies, and centered in science of public good to the government and researching to comprise environmental and earth processes. They in its laboratories manufactures reference soils and are the only one in the world selling reference soils with certified bioaccesibility, allowing the application of a UBM method to validation in laboratory, this reference material is named BGS 102.

Also faced to this problematic, is the option of soil remediation and phytoremediation, widely known, but they are problematic due to high costs and submitted to strict environmental regulations which must be fulfilled, thus, they become, in certain opportunities, so difficult since its technique must use own remediations to the ecosystem under study. Current solutions do not deeply solve this problematic is remarked due to the technique is not able to identify zones to be effectively remediated faced to others that likely do not. In this concern, organic remediations used or plants, do not distinguish what element is more or less toxic to human beings, only absorb those that are nutrient, then, might be applied to a while zone, encompassing wide extensions, becoming these techniques in extremely expensive.

On the other hand, the international environmental regulatory framework in force prevents the shipment of contaminated samples from countries which do not have certified analysis laboratory to countries having it and where soil studies are performed which are able to identify bioaccesibility of heavy metals like arsenic, cadmium and lead, among others, due to its clear adverse effects of the human being and its environmental interest since the same are duly regulated at national and international level.

This environmental problematic of soil contamination with impact in human health further has a heavy social impact since socio-environmental conflicts caused by soil contamination could be possible to demonstrate, showing an empowerment and supervisory role of communities in environmental aspects is indisputable.

On the other hand, there is in the international market, artificial stomaches available, particulaly DGM, "Dynamic Gastric Model" fabricated and patented by Martin Wickham, del Norwich Institute for Food Research, England, allowing the bioaccesibility estimation but the same are specially designed to simulate a contaminated food intake, thus, it is a complex design, of very high cost due to is able to simulate the destruction of a alimentary bolus contaminated by heavy metals, then this model an others are not a accessible solution to the environmental industry since the problematic under study is out of scope.

In relation to patent documents, it is possible to mention (Ecological Environmental Res. CT) related to a multi-channel simulator gastrointestinal system processing soil samples and requiring that an artificial digestive juice is prepared and added to samples at the multiple channel and requiring specific time and reaction conditions; then an extract is filtered and a heavy metal contaminant content is determined in an aqueous sample and is focused to risks in human health. CN108535046A (Ningbo Yinzhou Dazhifeng Indust Product Design Co Ltd) is related to an equipment to analyze samples having organic contaminants comprising a body having a cavity and cylindrical rotation with two rotating axis and slay out trays into a multiple reaction chamber.

CN101968480B (Univ Zhangzhou Normal) is related to a method to stimulate and detect microelement content in medicines which can be absorbed by the human body, wherein bionic digestive juices (simulated saliva, gastric juices, duodenal juice and bilis) in different digestion steps under a scheme as pharmaceutical doce form and obtaining a complete digestive juice; and simulating distribution and absorption conditions in stomach and intestine by a system of water-liposome distribution having a single layer with microelements, providing a new method to evaluate an effective dose of a complex of microelements to be absorbed by a body.

CN101726436A (Univ Zhejiang) is related to a method for extracting human body gastric flu.id simulated to absorb powder or soil heavy metals, comprising drying and finely milling a sample and adding an acetic acid solution to prepare a sample suspension, moving it into a water bath, regulating pH at 5.9-6.1, 3.9-4.1 and 1.9-2.1 using hydrochloric acid and carrying out preservation by heat and mechanical impact after each pH regulation; extracting and filtering to have a clear liquid and detecting heavy metal content in the same. This method simulates the main biochemical reactions and a powder or soil digestion dynamic in the human body and can provide support to evaluations of risks in human health.

KR101300445B (Shong Dong Hyung) is related to an autonomous continued operation "batch" reactor, in a gas during heavy metal analysis and before soil should be pre-treated. This batch is an integrated model more efficient and stable in structure.

CN106841460A (Hefei Zhihuilong Machinery Design Co Ltd) is related to a device to detect and analyze by an arrangement panel, fixed plates and "driver" arrangement.

CN104851346 (Univ Jinan) is related to an *in vitro* module-type simulator system to animal digestive tube and method associated comprising an *in vitro* simulating unit of at least animal digestive tube and completing by multi-way an intestinal simulation, a concrete and functional module to relatively independent tasks, a frame provided of installation multi-position, where module and re-cake are detachable, comprising a sensor and a mobile component or both. Each functional module has a control chip and is connected to a computer. The frame is equiped with an active USB concentrator. This USB is responsable of a single chip microcomputer in the functional module and powering and instructing a mutual transference to an upper part and lower computer. Multiple functional modules are integrated in a saving space frame in a laboratory and the module can be quickly replaced.

CN1013439476B (Beijing Munincipal Res Int Environment Prot) is related to a device and method to testing availability of heavy metals by simulation of human consumption characteristic, comprising the following steps: 1) pre-processing of soil sample, 2) configurating gastric juices of artificial simulation and intestinal juice and heating, 3) pre-treated soil sample and gastric juices of artificial simulation are added into an extraction flask to simultaneously regulating pH, 4) continuous tipping vibration, 5) after filtering with a ICP-MS membrane in stomach step, 6) after complemented a fresh gastric juice, intestinal juice of artificial simulation is added, and pH is regulated and fixed into the extraction chamber, 7) tipping vibration; 8) after ended filtration, membrane is changed and heavy metal is analyzed in human body intestine step by ICP-MS. 12 simultaneous samples can be analyzed and testing volume is huge, can be widely used in soils contaminated with heavy metals at a contaminated site.

CN109975524A (Jiangsu Provincial) is related to a testing device of heavy metal bioavailability and a method to simulate human digestion characteristics. Testing device comprising a vessel to simulate gastric digestion reaction; a module of feeding gastric digestion reaction connected to a vessel to simulate gastric digestive reaction and used to complement the needed liquid and gas to simulate a human gastric digestion in vessel, simulating thus human gastric juice secretion and an anaerobic digestive environment, a module of stirring located at the bottom of vessel to simulate gastric digestive reaction and used to agitate liquid into a vessel, simulating in that way a human stomach peristole; a module of temperature control connected to vessel to simulate a gastric digestive reaction and used to controlling liquid temperature into vessel, simulating thus a human body environment; a module to monitoring located into vessel to simulate reaction and used to monitoring temperature and pH liquid value into vessel; and a module capturing digestive reaction communicated with vessel to simulate a gastric digestive reaction and used to simulate gastric emptying and capturing liquid captured in vessel.

WO2006052742A (Smithkline Beecham Corp) is related to an apparatus and method to analyze the release of active agent(s) of pharmaceutical products and types, more accurately simulating the gastrointestinal tract conditions (GI) included forces applied to dose forms.

In summary, currently metal bioavailability to soil samples is calculate by several independient processes in a lab environment and in manual way, what makes the process so slow, upper 8 hours, and susceptible to errors to every of at least 7 steps, using recourses and large scale laboratory installation, not allowing a parallelism of measurements and requiring a specialist dedicated to a whole operation. The present light and portable gastrointestinal simulator module allowing to know an in real time and in situ heavy metal and metalloid bioaccesible concentration from soil samples, under automatized way, if desired, reducing at a maximum the specialist intervention, risks and chain errors and allowing a parallelism in measurements, non-synchronically depending on a single specialist. The present light and portable gastrointestinal simulator system allowing a cost transfer and sampling process saving, and support strategic decisions, in real time and short time, as difference as all the currently available today, which is performed at laboratory level using many instruments and apparatus delaying for months a result.

Then, faced to the social-economical and environmental problematic as described above, the present invention proposes a low cost, light and portable, gastrointestinal simulator system, that remotely and in real time quantify the bioaccesibility of heavy metals presents in samples of soil, such as mining soils, including tailings to perform studies of contaminated soils having realiable results. To then, a UBM method is integrated in a single technological equipment to quantify the bioaccesibility of heavy metal causing health damage, on-line and in real time, to facilitate the strategic decision making of companies belong to mining or environmental industry or the like, to ensure the protection of human health when exposed to these soils potentially contaminated such as neighboring populations to a tailings or contaminated land for residential use, and for faithful compliance with current regulations.

The present light and portable, gastrointestinal simulator modular system fulfilling an UBM (Unified BARGE Methodology) method and being able to remotely and in real time quantify, bioaccesibility of metals and metalloids selected from Fe, Zn, Cu and Pb, from a soil sample including tailings. Also allowing the identification of zones wherein soil remediation techniques should be strictly to apply and wherein a high metal or metalloid content causing damage to human beings, is demonstrated, and thus, the application of such remediation technique will become in more efficient and lower expensive.

### Breif Description of Drawings

**Figure 1** Scheme of the present light and portable, gastrointestinal simulator modular system.
**Figure 2** View of a whole 3D design of the bioreactor system.
**Figure 3** Views of flat projections of a whole 3D design of the bioreactor system.
**Figure 4****:** Views of isometric projections of a whole 3D design of the bioreactor system.
**Figure 5****:** Views in projections of the pre-mixing system.
**Figure 6****:** "end-over-end" spinning system and control of temperature.
**Figure 7****:** "end-over-end" spinning system and gear submodule.
**Figure 8****:** Gear submodule specification.
**Figure 9****:** Design of lower recirculation module.

### Breif Description of the Invention

An objective of the present invention is providing a method and light and portable gastrointestinal simulator system to quantify, remotely and in real time, bioaccesibility of metals and metalloids in a contaminated soil sample, including mining tailing soil, which could be accessible to human body under regulated conditions of human digestion (bioaccesibility) and determining risks to human health.

Another objective of the present invention is providing a light and portable system properly replicating or simulating conditions found in gastrointestinal system (GI).

Another objective of the present invention is also providing a light and portable system efficiently performing metal and metalloid quantification in a contaminated soil sample.

Another objective of the present invention is also providing a light and portable system performing in lower time and in situ, metal and metalloid quantification in contaminated soil samples.

These and other objectives, advantages and features of the present invention will be understood in the following description:
A light and portable, gastrointestinal modulator system remotely and in real time quantifying, bioaccesibility or quantity/concentration of metals under human digestion conditions, of metals and metalloids selected from zinc (Zn), lead (Pb), copper (Cu), iron (Fe), nickel (Ni); arsenic (As) and cadmium (Cd), in contaminated soil samples, including tailing or mining residues, comprising:
a) At least a upper pre-mix or pre-gastric module homogenizing and conditioning temperature in a mixture to be analyzed and further allows the preparation of each gastric or enzymatic fluid selected from saliva (S), gastric fluid (G), bile (B) and duodenal fluid (D) and its pre-mix having a soil sample, comprising:
   a.1) at least one first mixing receiving mean receiving such fluids S and G and such soil sample, wherein such first mixing receiving mean is of an inert material, including stainless steel or glass, preferably glass,
   a.2) at least one first stirring mean, at least one first sensor of stirring rate, at least one first regulator mean of stirring rate and at least one first controlling mean of such stirring rate, located in such at least first mean receiving a sample, allowing to keep a stirring rate to such first stirring mean allowing to achieve 90% homogeneity;
   a.3) at least one first temperature sensor, at least a first temperature regulator mean and at least one first temperature controlling mean, located in such at least first mean receiving a sample to allow to keep temperature inside of such at least one first mean receiving a mixture at a ranging from 35 to 39ºC, preferably 37°C,
   a.4) at least a first pH sensor mean, at least a first pH regulator mean and at least a first controlling pH mean, located in such at least first mean receiving a sample, allowing:
      a.4.1) regulating pH value ranging from 6.0 to 7.0 when such first vessel initially receives such fluid S, in isolation; or
      a.4.2) regulating a pH value ranging from 1.0 to 1.2 when such first vessel initially receiving such fluid G in isolation
      a.4.3) regulating a pH value ranging from 1.15 to 1.25, preferably a pH value of 1.2, when such first vessel receives such soil mixture in addition to fluid S and fluid G,
   a.5) at least a second mixing receiving mean receiving such fluids B and D, and operating in parallel to such at least first mixing receiving mean, and wherein such first mixing receiving mean is of an inert material, including stainless steel or glass, preferably glass;
   a.6) at least a second pH sensor mean, at least a second pH regulator mean and at least a second controlling pH mean located in such at least one second mean of receiving a sample, allowing
      a.6.1) regulating pH value ranging from 7.8 to 8.2 when such first vessel initially receiving such fluid B in isolation; or
      a.6.2) regulating pH value ranging from 7.2 to 7.6 when such first vessel initially receiving such fluid D in isolation
   a.7) at least one second temperature sensor, at least one second temperature regulator mean and at least one second temperature controlling means, located in such at least second means receiving a sample, allowing to keep temperature inside of such at least second mix receiving mean at a ranging from 35 to 39ºC, preferably at 37°C,
   a.8) at least a second stirring mean, at least a second sensor of stirring rate, at least a second regulator mean of such stirring rate and at least a second controlling mean of such stirring rate, located in such at least second mean receiving a sample, allowing to keep stirring rate to such second stirring mean allowing to achieve a 90% homogeneity, and
   a.9) at least an interface of display screen showing information of such at least one first and second temperature sensors, such first and second pH sensors and such at least first and second stirring rate sensors, and
   wherein such at least one first and second temperature controlling means allowing a manual or automatized action in such first and second temperature regulating means to to keep temperature inside of such at least first and second means receiving a mixture, at 37ºC,
   wherein such at least one first and second stirring means are selected from at least one magnetic stirring mean, even more preferably, from at least a bladeless ferromagnetic stirring mean,
   wherein such at least one first and second temperature sensor means are preferably selected from infrared temperature sensors,
   wherein such at least first and second temperature regulating means are selected from at least two fan heaters having asynchronous activation, and
b) at least an intermediate mixing or gastric and gastrointestinal simulator module, comprising:
   b.1) at least a bioreactor, preferably stainless steel receiving such mixture of such soil sample added to fluid S plus fluid G or a mixture of such soil sample added to fluid S plus fluid G plus fluid B plus fluid D,
   b.2) at least a third stirring mean selected from an "end-over-end"-type agitator allowing a 360º spin located in such at least one bioreactor, comprising at least one third stirring rate sensor, at least a third regulator mean of such stirring rate and at least a third stepper controlling mean of such stirring rate, allowing that over 50% homogeneity of mixture is achieved, preferably 90%, and at least one third temperature sensor, at least one third temperature regulator mean and at least third temperature controlling mean, preferably, a pneumatic temperature controlling mean,
   b.3) at least one first supporting base mean to each rotation axis of such at least third stirring mean, and at least one mounted gear mean over side support bearing media, allowing a friction reduction against the internal wall of such at least one bioreactor, achieving mechanical balance, giving support, having low resistance to the movement, and moving such at least one bioreactor, and at least one third rotatory rate sensor mean to such third at least one third stirring mean, at least one third rotatory rate regulator mean to such at least stirring mean and at least a third rotatory rate controlling mean to such at least third stirring mean,
   b.4) at least a third temperature sensor, at least a third temperature regulator mean and at least a third temperature controlling mean, located in such at least one bioreactor allowing to keep temperature inside of such at least one bioreactor at a ranging from 35 to 39ºC, preferably 37°C,
   b.5) at least one first pH sensor, at least a first pH regulator and at least one pH controlling mean allowing the mixture inside to such at least one bioreactor either to,
   b.5.1) a pH value ranging from 1.15 to 1.25 if the mixture incoming to such bioreactor corresponds to such mixture of such soil sample plus fluid S plus fluid G, and a pH value ranging from 5.8 to 6.8 if the mixture incoming to such bioreactor corresponds to such mixture of such soil sample plus fluid S plus fluid G plus fluid B plus fluid D,
   b.5.2) a pH value equal or lower 1.5 if the mixture incoming of such bioreactor corresponds to such mixture of such soil sample plus fluid S plus fluid G and a pH value ranging from 5 to 8 if output mixture of such bioreactor corresponds to such mixture of such soil sample plus fluid S plus fluid G plus fluid B plus fluid D,
   b.6) at least a centrifugation mean allowing a centrifugation to a biochemical extract contained in such at least one bioreactor,
   b.7) at least one equipment to quantify metal or metalloids selected from copper (Cu), lead (Pb), Zinc (Zn) and Iron (Fe), Nickel (Ni); Arsenic (As) and Cadmium (Cd), preferably, selected from one of: a photometer, a thermo-reactor, a microwave or a Parr pump or a mobile quantification mean selected from a X-ray or XRF fluorescence gun or a heavy metal quantification mean selected from plasma mass spectroscopy (ICP) or atomic absorption spectroscopy;
   wherein such at least one third temperature sensor is selected from an infrared sensor and such third temperature regulator mean is selected from the combination of at least a fan and at least an electric resistance;
   wherein such at least third temperature sensor measures temperature inside and in vicinity of such at least one bioreactor,
   wherein such stirring rate regulator mean in such at least one bioreactor is selected from a stepper motor, and
c) at least one low recirculation module comprising:
   c.1) at least one third receiving mean receiving by empty or gravity discharge, biochemical extract resulting from the mixing or intermediate module,
   c.2) at least one impelling mean including a peristaltic pump allowing recirculate such biochemical extract forward a pre-mix or upper module, and
d) at least a stirring rate monitoring or data processing mean, in each one of such at least first, second and third stirring means; temperature, in each one of such at least first, second and third receiving means and bioreactor; and pH, in each one of such at least first, second and third receiving means and bioreactor, comprising at least one data center remotely receiving such stirring rate data, temperature and pH from such at least one first, second and third stirring rate, temperature and pH sensors of such pre-mix module and intermediate module, wherein such at least one data center comprising at least one database wherein such data received from such at least one first, second and third stirring rate, and at least one processor processing such received data and sending at least one output signal in response to such at least first, second and third stirring rate controlling means, temperature controlling means and pH controlling means, after compared such received data against stored, referential or pre-set data, in such at least one database, enabling manual or automatized actuation, in one or more of such at least one first, second and third rotatory rate controlling means, pH controlling means or temperature controlling means if confirmed a difference between such received data and stored referential or pre-set data.

Such fluids S and G are fed in such at least one first mean of receiving sample and such fluids B and D are fed in such at least second mean receiving samples by gates located at the upper part of such at least upper and second means receiving sample.

Optionally, the present modular system comprising emitting signals being visual, sonorous or both, warning to a user that enough time has passed to achieve 50% homogeneity in an intermediate module, preferably, 90% homogeneity.

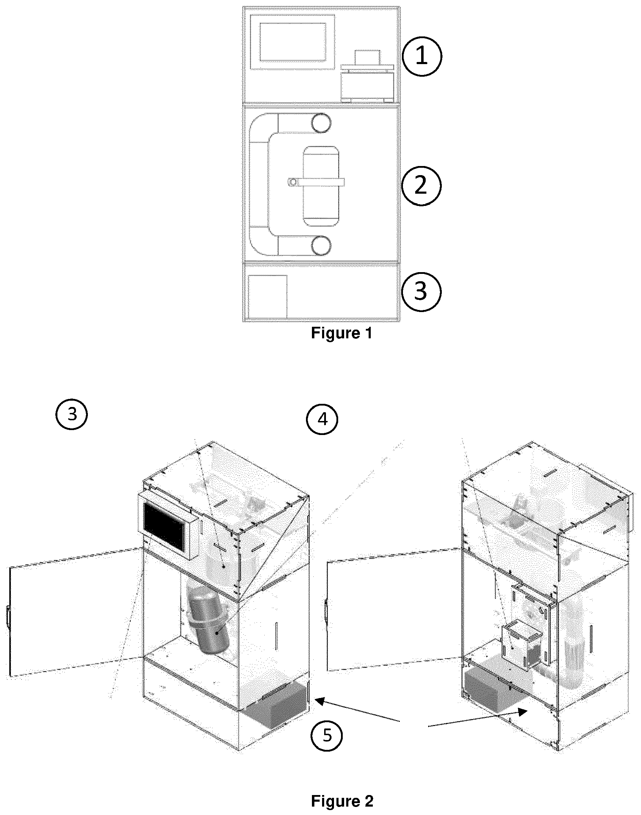

Figure 1 shows the present modular system, wherein each module is identified with numerals 1, 2 and 3. Module 1 (pre-mixing module) corresponds to a pre-mixing module incorporating a dual magnetic stirring system which also is useful as support of the monitoring system of process variables. Module 2 (intermediate module) mainly showing an "end-over-end"-type bioreactor, composed by a rotatory 360 system, controlling step by step and also incorporating temperature control means selected from pneumatic temperature controlling means. Module 3 (recirculation module) mainly showing impeller recirculation means, preferably peristaltic pumps.

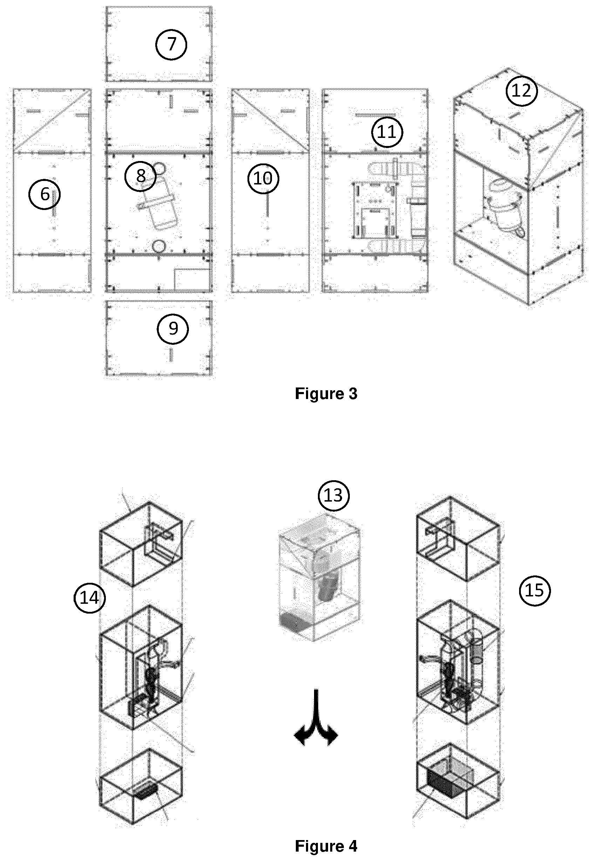

As showed by Figure 1, the present system is designed as a system of boxes or cubes able to assembly therebetween, as showed in the tridimensional projective diagram illustrated in Figures 2, 3 and 4.

Any of the controlling means comprising a platform based on a microprocessor functioning with a master system in edge computing" modality which is directly connecting to control subsystems based on microcontrolling platforms. Values/measurements sent by sensor means collected from each module, temperature, pH and stirring/rotating rate, are processed, allowing a response to acting on the actuators of such regulator means of any of such modules, and also, the same are sent to a database in "cloud computing" modality and used to implement a monitoring web system, a "back-end" system, to process in course, "front-end".

Control system is composed by a platform based on a microprocessor, which functions as master system in "edge computing" modality which is directly connected to control subsystems in platforms having microcontrollers. Master controller records different captured variable by sensor means, which are processed by control algorithms to acting on actuators of each of modules. Together with the above, recorded variables are informed in a database in "cloud computing" modality, being useful as input to a "back-end" system to a monitoring web service to a process in course ("front-end").

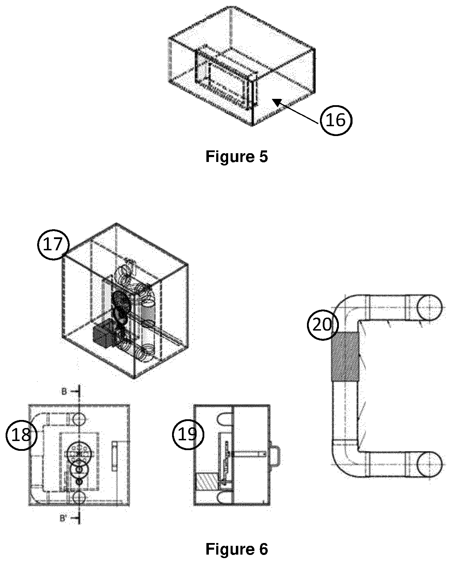

Figures showing such magnetic means of pre-mix module, allowing a stirring of the mixture inside the first and second receiving mean by a ferromagnetic stirring mean without blades, conferring a constant spinning and allowing a mixture achieving a 90% homogeneity. Also such temperature and pH regulation and control means are showed, allowing a fluid pre-mix with soil samples. See Figure 5.

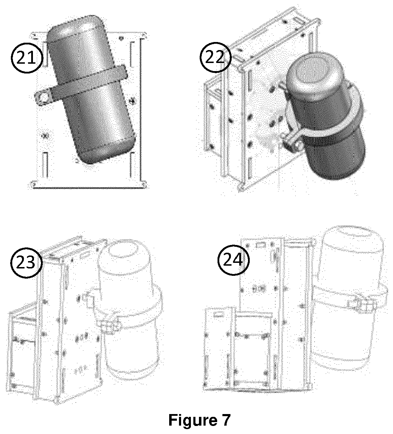

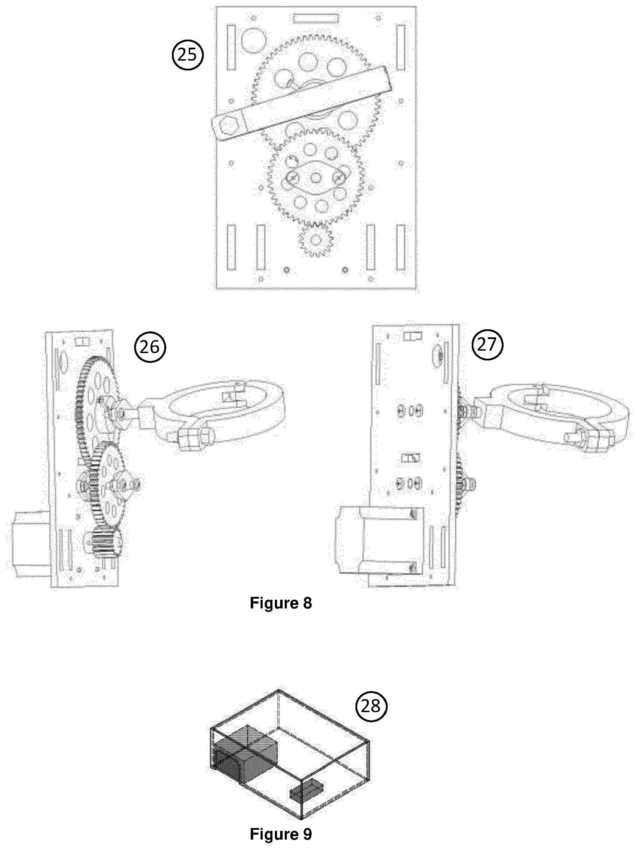

Figures also show an intermediate module comprising such "end-over-end" biorreactor wherein chemical reactions occur comprising stirring means with an "end-over-end"-type spinning and operating ranging from 25 to 30 RPM, preferably 29 RPM and an agitation rate regulated by a stepper motor. Also, such stirring mean supported in gear means having different axis mounted over a bearing series having lateral support and such stirring/spinning rate sensor mean is showed. Also, such temperature regulating means inside such bioreactor is showed, comprising a fan and an electric resistance and temperature sensor means selected from an infrared sensor to measure focal and environmental temperature of such bioreactor while spinning. See Figures 6, 7 and 8. Figure 6 shows a pneumatic circulation system for controlling temperature. Additionally, Figure 7 shows a complete spinning of "end-over-end" spinning means and a container box to the stepper motor. Figure 8 showing "end-over-end" spinning means comprising a gear set as reducer and amplifier box to torque force of stepper, used to regulate along the whole time an exact rate to which vessel having reagents is intended to rotate. Also, the reagent empty and filling vessel position is needed to control. A calculate of radius and teeth number for each gear has direct relation with the required volume inside bioreactor.

Figure 9 shows a recirculation module and mainly an impeller mean, peristaltic pump, enabling a recirculation to pre-mixing module to repeat a process cycle, if necessary.

A test having tailing soil abandoned in city is performed, the process of taking sample and its handling was made saving good practices allowing to ensure reliability, traceability, safety, and reproducibility as much to a process development as to obtain results.

The present method to remotely and in real time quantify, metal and metalloid bioaccesibility in a contaminated soil sample:
a) preparing organic and enzymatic fluids selected from (S), bile (B), gastric fluid (G) and duodenal fluid (D), as the following steps:
   a.1) preparing such organic fluid S, adding water including water ultra-pure, under stirring up to achieving a 90% homogeneity and maintaining a temperature at 28ºC, in a volume/volume ratio 3/10, and adding and oxidant agent, preferably, NaOH, to regulate pH value ranging from 6.0 to 7.0, if needed, and then gradually heating mixture up to achieving 37ºC;
   a.2) preparing such organic fluid G, up to achieving a 90% homogeneity and maintaining temperatura at 28ºC, in a volume/volume ratio 37/80, and adding an acid reducing agent, preferably, hydrochloric acid to regulate pH value ranging from 1.0 to 1.2, if needed, and then gradually heating mixture up to achieving 37ºC;
   a.3) preparing such organic fluid B, adding water, preferably ultra-pure water under stirring up to achieving a 90% homogeneity and maintaining temperature at 28ºC, in a volume/volume ratio 3/10, and adding an acid reducing agent, preferably, hydrochloric acid to regulate pH value ranging from 7.8 to 8.2, if needed, and then gradually heating up to achieving 37ºC; and
   a.4) preparing such organic fluid D, adding water, preferably ultra-pure water under stirring up to achieving a 90% homogeneity 90% and maintaining temperature at 28ºC, in a volume/volume ratio 37/60, and adding an acid reducing agent, preferably hydrochloric acid to regulate pH value ranging from 7.2 to 7.6, if needed, and then gradually heating mixture up to achieving 37ºC;
b) preparing a contaminated soil sample preferably, abandoned tailing soil in city, drying at a temperature ranging from 40 to 80ºC, preferably 40ºC up to achieving 0% humidity and subsequently screening up to a size lower 250 micrometers,
c) mixing such resulting soil sample of step b) with such resulting fluid S of step a.1) and such resulting fluid G of step a.2) and let reacting into bioreactor by a while, and then adding the resulting reacted mixture into reactor with resulting fluid B of step a.3) and such resulting fluid D of step a.4) maintaining a temperature of such bioreactor ranging from 37 to 39ºC, preferably 37°C, and where such mixtures are performed under stirring up to obtaining 50% homogeneity, preferably 90% by the following sub-steps:
   c.1) mixing such contaminated soil sample as step b) with fluid S prepared as step a.1) in a weight/volume ratio of such soil sample to such fluid S (mg/ml) from 100 to 15, under stirring,
   c.2) adding to such resulting mixture of step c.1), fluid G prepared as step a.2), in a volume to volume ratio S: G of 2: 3, under stirring and regulating pH at a value ranging from 1.15 to 1.25, preferably, a pH value 1.2, and let reacting by a time of at least 1 hour;
   c.3) adding to such resulting mixture of c.2), fluid D prepared as step a.3), in a volume to volume ratio S: G: D of 2: 3: 6, under stirring;
   c.4) adding to such resulting mixture of step c.3), fluid B prepared as step a.4), in a volume to volume ratio S: G: D: B of 2: 3: 6: 2, under stirring and regulating pH value ranging from 5.8 to 6.8, preferably, a pH value 6.3, and let reacting by at least 4 hours, and
d) centrifugating resulting mixtures of gastric step or step c.2) or resulting of gastrointestinal step or step c.4) and separating supernadant to after adding an oxidant agent preferably, nitric acid, preferably nitric acid 67%, and preparing dilutions with mixtures, wherein such dilutions prepared are ranging from 1:200 to 1:10, preferably dilutions from 1:10, 1:100 and 1:200, and one from:
e) eliminating organic material contained in such dilutions of step d) submitting to an oxidant acid medium or a metal disintegration digestion such dilution, at 120°C, or by acid digestion by microwave,

wherein prior to carry out such digestion of metal disintegration pH is regulated with oxidant agent, f) separately quantifying such metal or metalloid selected from copper (Cu), iron (Fe), Zinc (Zn) and lead (Pb), Nickel (Ni), Cadmium (Cd) and Arsenic (As), from resulting samples of step e), in
a photometer, a thermoreactor, a microwave or a Parr pump or mobile quantification mean selected from a ray-X or XRF fluorescence gun or heavy metal quantification selected from plasma mass spectroscopy (ICP).

Optionally, steps a) and c) are repeated as much times as considered as needed, wherein fluids S, B, D, G prepared are mixed with resulting sample of step c) above of each repeating cycle.

Optionally, the present modular system comprising emitting signals auditory, visual or both, warning to the user that enough time has passed to achieve a mixture homogeneity over 50%, preferably 90% either when sample is mixed with S and G or when sample is mixed with S, G, B and D. Also, a warning is considered when pre-set thermal levels are exceeded.

Being the time enough up to achieve a 50% homogeneity in the intermediate module of 30 minutes. When such soil sample is mixed with S and G up to achieving a 90% homogeneity in the same module when such soil sample is mixed with S and G, time is approx. is 1 hour.

Being the time enough up to achieve a 90% homogeneity in the intermediate module when such soil sample is mixed with S, G, B and D of 2 hours, while when such soil mixture is mixed with S, G, By D, a 90% homogeneity is achieved in a time approx. of 4 hours.

Optionally at the end of step c) 1 to 2 ml concentrated nitric acid (HNOs) is added to fixing a pH value ranging from 5 to 8, in case if desired, preserving such sample.

Preferably, step e) comprising regulating pH value with an oxidant agent selected as stated below, depending of metal to quantify:
e.1) regulating pH value ranging from 6 to 7, preferably with NaOH and HCl, previously quantifying Fe from such dilution,
e.2) regulating pH value ranging from 6 to 7, preferably with diluted NH₃ and HNO₃, previously to quantify Pb from such dilution,
e.3) regulating pH value ranging from 6 to 7, preferably with NaOH and H₂SO₄, previously to quantify one of Zn, Cu, Ni, Cd or As from such dilution.

Preferably, such digestion of metal disintegration of step e) carry out to a thermoreactor having reagents which are added as metal to be quantified and as manufacturer instructions.

Preferably, if quantification step e) is carried out in a photometer, pH of such mixture is regulated as metal to be quantified from such dilution by using of oxidant agents selected as stated below:
f.1) regulating pH value ranging from 3 to 6 with diluted NH₃ and HNOs to quantify Pb from such dilution,
f.2) regulating pH value ranging from 1 to 10 NaOH and HCl to quantify Fe from such dilution,
f.3) regulating pH value ranging from 3 to 11 with NaOH and H₂SO₄ to quantify one of Zn, Cu, Ni or Cd from such dilution.

Preferably, Arsenic (As) is quantified submitting such dilution to a photometric arsenic test as manufacturer instructions wherein previously pH of such dilution value is regulated ranging from 0 to 13.

### Examples

Before submitting to analysis and quantifying metal and metalloid quantity of each sample, good practices of laboratory analysis need to be fulfilled and pH sensors must be calibrated to give reliability to the analysis process, which should be significantly considered. Similarly good practices to laboratory analysis must be assured when performed a sampling.

Also soil sample to be analyzed should be previously submitted to drying at a temperature ranging from 40°C to 80°C, preferably, 40°C, up to 0% moisture content, which should be significantly considered, taking a time of 6 to 8 hours, and should be previously screened at a size lower than 250 micrometers.

### Example 1

8,15 g tailing soil sample (indey 106) having features as stated in table 2, is added to 368 mg saliva organic fluid (S) previously dissolved in 120 mL ultra-pure water, under controlled stirring from 500 to 750 rpm and heating up to achieving 28°C and adding with micropipette 0.22 ml NaOH (1M), slightly regulating pH to isolated fluid S and controlling pH at range from 6 to 7. Mixture of organic fluid S and soil sample under stirring from 500 to 750 rpm for 10 to 15 seconds and slightly heating up to achieving 37°C. Then, 2051 mg fluid gastric (G) previously prepared dissolved in 185 ml ultra pure water, under controlled stirring and heating up to 28°C is added and added with micropipette 1.53 mL HCl (37%), slightly regulating pH and controlling pH of fluid G ranging from 1.0 to 1.2. Mixture with organic fluid G is performed under controlled stirring ranging from 500 to 750 rpm for 10 seconds and slightly heating up to achieving 37°C. Resulting sampling prepared as mentioned, adjusted at a pH range from 1.15 to 1.25, is income to a bioreactor module of the present system and is agitated at 29 rpm for 1 hour, at the end is confirmed that a pH value lower 1.5 and is centrifugated at 4500 g for 15 minutes to then removing supernadant and waiting for its cooling at room temperature. Resulting extract is prepared by acid attack or acid digestion before obtaining photometer readings to identify presence of lead (Pb), a metal which is stated by several literature as one retained in gastric step. Acid digestion and high temperature (300°C) and applied pressure (2500 psi), allowing the destruction of all organic material, and thus, facilitating the generation of proper measurements associated to metals.

**Table 1 Tailing and mining soil characteristics**

| | |
|---|---|
| IDQ | 1062 - Sample 1 |
| Mining Recourse | GOLD-COPPER-SILVER |
| Origin | MURO |
| Coord. N | 6651618 |
| Coord. E | 299398 |
| Cu (g/t) | 808 |
| Ni (g/t) | 10 |
| Zn (g/t) | 860 |
| Pb (g/t) | 1017 |
| As (g/t) | 162.83 |

To eliminate organic material interference presents into the extract is performed a previous extract digestion in an oxidant acid medium at 120°C for 1 hour, to oxidate organic material and let metal free for its determination by photometry, requiring extract dilution and adjusting pH in sample. Dilutions 1:10; 1:100; 1:200 allow metal detection. Before performing an organic material destruction, sample is stabilized at pH values ranging from 5 to 8 to assure procedure effectiveness. Oxidant acid medium can be selected from NaOH (1M), HNO₃ (0.1 M), H₂SO₄ (0.5 M), HCl (3%) or diluted ammonia. Taking photometric measurements to iron adjusting pH to dilutions at values ranging from 6 to 7 with NaOH or HCl, to lead adjusting pH to dilutions at values ranging from 6 to 7 with NH₃ or HNO₃ and to other metals adjusting pH to dilutions at values ranging from 6 to 7 with NaOH or H₂SO₄.

To eliminate organic load/material, a thermoreactor is used to pre-heating at 120°C and a digestive reagent set: digestive acid as reducing agent (99.7% pure sulfuric acid), and an oxidant agent, a mixture of potassium persulfate and sodium nitrate, which let react sample with reagents for 1 hour to then let cooling at room temperature. Reagent 3 is added, sodium hydroxide, NaOH, as additional reagents to adjusting pH of sample, only to measuring iron. To the other ones metals a portable photometer is used and manufacturer instructions are followed.

Alternatively, and to confirm or obtaining values when photometer measurement is failed, an acid Parr digestion can be carried out, counting with the inclusion of pressure sealing vessels as advantage, a quick procedure to sample dissolution. Also a measuring supported in the use of commercial microwave can be carried out. A convenient media to keep strong mineral acid or alkali at temperatures very high to normal boiling points is provided, allowing digestion acceleration by use of strong acids as HF or aqua regia, generating a vigorous chemical action allowing reducing time associated to performing analytical procedures.

In performed test, an acid agent and another reductor were added to the sample at equal ratios, to 4 ml sample 4 mL de HNO₃ (65%) and 4 mL de H₂O₂ 30% is added, and containers were closed under pressure. Depending on tailing or residue to treat these quantities can be modified at a ratio 1: 1: 0.5 volume. Generally, 3 min were used, at low power ranging from 300 to 450 Watts, preferably 400 Watts, and with caution that sample volume including mixture of acid agent and reducing agent do not exceed 40% final volume Parr pump.

Chemical analysis of elements in solution by photometric method is a classic technique to analysis of different types of water such as superficial, drinking, residual, etc. To this particular case of fluids used in a bioaccesibility procedure, is more complex to apply photometric techniques due to a wide range of compounds and salts, much of them of organic nature, composing each fluid. In the present invention, reagents are added to samples containing metal and metalloids to determine and introduced into a tray, causing a coloring having an intensity which is proportional at metal or metalloid concentration in sample. By means of a sample vessel media, a beam light is passed which is partially absorbed by sample, non-absorbed part of such beam light is recorded by photometer and converted into a signal to concentration data. To each metal or metalloid a specific reactive should be used, applied to a specific quantity over an adjusted pH dilution and added to a tool calibration as defined for manufacture, allowing directly obtaining metal or metalloid concentration.

To determine measurements with lead, two measurements should be performed since first measurement is related to free Pb plus some other impurities caused by a reagent reaction. A measurement is related then with sample while another measurement is related to impurity of this chemical test, calcium, and which is over 100 ppm Ca⁺. To measuring impurity then ammonia (NH₃) is added which after fully dissolved, is used to substracting impurity, for the most part.

To determine total copper concentration, an acid mixture is required to dissociate stable copper compounds and complexes. To avoid passing a range of instrument detection (0.05 - 8 mg/L), sample should be diluted, preferably, in a dilution ranging from 1 to 10, up to a maximum from 10 to 100) directly with deionized water and pH adjusted with diluted NaOH or diluted H₂SO₄ before performing a measurement and adding a corresponding reagent to metal test is necessary. To lead is used diluted NH₃ and HNO₃. To iron is used NaOH and HCl. To Zinc, Cobre, Nickel and Cadmium NaOH and H₂SO₄ is used and to arsenic test the following methodology should be proceeded:
Arsenic test is performed as established by the photometric Method Test Spectroquant^{®}, code 1017470001 requiring a volume of sample of 20 ml and using different reagents. Then, following the manufacturer instructions a measure was performed and the resulting extract was submitted to a direct photometer reading. This test does not require that sample is treated with a previous acid digestion since process itself includes a reducing acid treatment.

To determine Zinc concentration a longer reaction time than usual is required to carry out measurements comparable with other metals and metalloids, 15 minutes. Reagents are according to instrument manufacturer's statements. Generally, reaction color remains intact for 30 minutes, decaying its concentration at 5% in relation to an initial measurement value, at 60 minutes. pH is adjusted to values of 3 to 10 with diluted NaOH or diluted H₂SO₄. Range of detection of sample is 0.2 to 5 mg/L.

Due to nitrate anions and coming from sands such as silicon oxide together with sulfates produce measurement interferences over 1000 ppm, a sample prepared at a temperature ranging from 10 to 35°C with regulated pH at a value ranging from 4 to 10 is added; and reagents stated by the measurement instrument manufacturer (photometer) are used.

To iron measurement, generally soils having a high concentration of this element due to its natural abundance as should be present, and thus, sample should be diluted many tomes with distilled water to be within the photometer detection range (0.05 to 4 mg/L). Dilutions can be selected from 10, 100 or 20, preferably. pH can be adjusted to a value ranging 1 to 10, preferably pH is adjusted with NaOH y HCl. Reagents suggested by the measurement device's (photometer) manufacturer are used.

Gastric step data is summarized in table 2:

**Table 2**

| | |
|---|---|
| Tailing mass under study (g) | 8.1569 |
| Final volume inside bioreactor (L) | 0.305 |
| pH Fluid S | 6.56 |
| pH Fluid G | 1.1 |
| Temperature (°C) | 37.3 |
| Adjustment with HCl 37% | 2 mL + 12 drops |
| Output pH | 1.21 |

To analysis of sample 2 under study Indey 106 tailing to gastric step was performed using the same procedure than those one describe above to gastric step of metal quantification under study:

**Table 3 Results of gastric step example indey 106**

| ELEMENT | NOVA Conc. Bioaccesibility ppm | Laboratory Conc. Bioaccesibility ppm | PXRF CONC.TOTA L ppm | % Bioaccesibility Bioaccesible concentration of prototype (invention)/total concentration |
|---|---|---|---|---|
| Pb | 94.22 | 109 | 258 | 37 |
| Zn | 377.51 | 183 | 463 | 82 |
| Fe | 2485.57 | 2478 | 58465 | 4 |
| Ni | 21.3 | 8,8 | <LOD | Nd |
| Cd | 0.45 | 2,1 | <LOD | Nd |
| Cu | 1513.77 | 1494 | 1796 | 84 |
| As | 5.15 | Nd | 64 | 8 |

Tables 4 and 5 show results obtained of bioaccesible concentration to the system of the present invention to 4 metals as example, and with 6 repetitions as evidence of validation to system comparing results with those one obtained in Laboratory.

**Table 4: Independent readings/measurements of bioaccesible concentration in gastric step determined by Atomic Absorption Spectroscopy (EAA) of a tailing sample, sample 2 (indey 106), by the present system versus laboratory**

| Repetition | Sample | ppm Cu | ppm Fe | ppm Pb | ppm Zn | ppm Cd | ppm Ni | Origin |
|---|---|---|---|---|---|---|---|---|
| I7 | Indey 106 | 1409 | 2464 | 98 | 185 | 2.1 | 8.8 | Prototype |
| I9c | Indey 106 | 1322 | 2610 | 95 | 174 | 2.1 | 32 | Prototype |
| 112 | Indey 106 | 1384 | 2406 | 95 | 179 | 2.1 | 8.8 | Prototype |
| 113 | Indey 106 | 1398 | 2288 | 98 | 165 | 2.1 | 10.4 | Prototype |
| In2 | Indey 106 | 1508 | 2402 | 103 | 180 | 1.9 | 5.7 | Laboratory |
| In6 | Indey 106 | 1349 | 2271 | 92 | 173 | 2,0 | 6.4 | Laboratory |

Table N°5 confidence intervals (I. C) to performance of the present system (Extraction Procedure) in step 1 of tailing Indey 106 to laboratory procedure and prototype (invention, SD is standard deviation, CV is variation coefficient percent)

**Table 5**

| | | **ppm Cu** | **ppm Fe** | **ppm Pb** | **ppm Zn** |
|---|---|---|---|---|---|
| Prototype | Media | 1373 | 2449 | 97 | 174 |
| | SD | 33 | 270 | 4.3 | 7.9 |
| | CV% | 2.4 | 1.1 | 4.4 | 4.5 |
| | IC | 1342-1404 | 2188-2710 | 93-101 | 167-182 |
| Laboratory | Media | 1438 | 1855 | 96 | 169 |
| | SD | 61 | 352 | 7.5 | 7.3 |
| | CV% | 4.3 | 19 | 7.8 | 4.3 |
| | IC | 1379-1498 | 1516-2195 | 89-103 | 162-176 |

As validation of reading system table 6 shows measurement by photometry of treated samples with exception of arsenic which does not require this preparation:

**Table 6 shows digestion acid assays results in gastric step. Relative errors of photometry reading performance with acid digestion vs EAA in sample 2.**

| Metal | Chemical Lab/AA value | Average value measured photometer/Acid Dig. ppm | Absolute Error = experimental Value - actual value | Relative Error = Absolute Error/Actual value) *100 |
|---|---|---|---|---|
| Copper | 1494 | 1513.77 | 20 | 1% |
| Iron | 2478 | 2485.57 | 7 | 0% |
| Lead | 109 | 92.22 | -17 | -15% |
| Zinc | 183 | 377.51 | 195 | 107% |
| Cadmio | 2 | 0.5 | -2 | -78% |
| Nickel | 9 | 21.3 | 13 | 142% |
| Arsenic | < 5 | 5 | Nd | Nd |

A detailed procedure to gastrointestinal step mainly searching bioaccesible arsenic is incorporated in the following late phases at the end of gastric step as explained above.

At this step a phase having 4 hours is added into bioreactor wherein organic fluids selected from bile and duodenal (B, D) are added according to the following protocol:
1. Preparing organic fluids B and D while gastric step is under process into bioreactor,
   a) Fluid D is added into a vessel, adding 370 mL water for analysis and stirring at a temperature up to 28°C,
   b) Fluid B is added into a vessel, adding 120 mL water for analysis, stirring up to dissolution and heating at 28°C.
   c) Adding 22 µL HCl to fluid B and 67µL HCl to fluid D.
   d) Regulating slightly temperature up to 37°C in both fluids.
   e) Verifying that each fluid is within a range of pH, to fluid B pH = 7.8 and 8.2 and fluid D pH = 7.2 and 7.6.
2. Ending the first hour of gastric step, a checking should be made to a pH lower 1.5 and solution B is added to a bottle containing extract of prior step.
3. Subsequently solution of fluid D is added to a bottle.
4. Agitating mixture and verifying pH is within range 5.8 and 6.8. If not be in this range pH is adjusted with NaOH 1M or HCl 37%
5. Once adjusted pH range, mixture is stirring for 4 hours at 29 RPM (range can be between 24-29 RPM)
6. Centrifugating at 4500g for 15 minutes.
7. Removing supernadant and let cooling at room temperature to refrigerate while an acid digestive system is prepared and its oxidant and reducing agents.
8. Preparing an extract to photometry measurements: Before performing measurements sample should be prepared while acid attack preferably using Crack Set de Merck or acid digestion by microwave. This process can last for 4 hours
9. Performing measurement, including arsenic which is proposed by scientific literature as a retainable compound at the gastrointestinal step.

Data to the elaborating process to gastrointestinal step is summarized in Table 7.

**Table 7:**

| | |
|---|---|
| Volume added inside Bioreactor (L) | 0.490 |
| Final volumen inside Bioreactor (L) | 0.795 |
| pH range Fluid B | 7.8-8.2 |
| pH Range Fluid D | 7.2-7.6 |
| Temperature (°C) | 37 ± 2°C |
| Adjustment NaOH | 3 mL + 12 drops |
| pH range entrance | 5.8-6,8 |
| Output pH | (only include pH) |

Following, results obtained to tailing sample 2 under study Indey 106 are showed to arsenic in both steps as conclusion to a whole operation of the system of present invention:

**Table 8**

| INDEY 106 | ARSENIC TEST NOVA | TOTAL CONC. | % Bioaccesibility |
|---|---|---|---|
| Sample 2 | Bioaccesible Conc. ppm | XRF ppm | Bioaccesibility concentration of the prototype/total concentration |
| STEP 2 | 46.05 | 64 | 72% |
| STEP 1 | 5.15 | 64 | 8% |

Following as showed results obtained of tailing sample 2 in study Indey 106 to lead in both steps as conclusion to the complete operation of the system of invention:

**Table 9**

| INDEY 106 | LEAD TEST NOVA | TOTAL CONC. | % Bioaccesibilty |
|---|---|---|---|
| Sample 2 | Conc. Bioaccesibility ppm | XRF Ppm | Bioaccesibility of prototype/total concentration |
| STEP 1 | 94.22 | 258 | 37% |
| STEP 2 | 19 | 258 | 7% |

Other results obtained to other mining residue samples and following the same conditions, steps and system of the invention is showed in tables 10 and 11 below:

**Table 10: Gastric Step (Indey 31)**

| | NOVA | Laboratorio | PXRF | Bioaccesibility |
|---|---|---|---|---|
| | Bioaccesibility Concentration | Bioaccesibility Concentration | Total Concentration | % |
| Element | ppm | ppm | ppm | |
| Pb | 747 | 613 | 692 | 89% |
| Zn | 6906 | 377 | 438 | 86% |
| Fe | 21279 | 23183 | 54929 | 42% |
| Ni | 821 | 1005 | <LOD | Nd |
| Cd | 485 | 0 | <LOD | Nd |
| Cu | 4 | 721 | 1899 | 38% |
| As | 30.71 | 35 | 103 | 34% |
| Pb | 0 | 41 | 692 | 6% |
| Zn | 109 | 321 | 438 | 73% |
| Fe | 2141 | 612 | 54929 | 1% |
| Ni | 1752 | 48 | <LOD | Nd |
| Cd | 10 | 0 | <LOD | Nd |
| Cu | 39 | 362 | 1899 | 19% |
| As | 16.21 | Nd | 103 | 16% |

These results show that quantifying total heavy metal concentrations and bioaccesibility in situ is possible.

While present invention has been describe doing reference to exemplary embodiments, a skilled person in the art can perform one or more changes or adaptations which can be used within the scope of this invention to correspond with mere elements of equivalence or substitution to the ones described herein. Present invention is not limited to descriptions and examples as described above.

## Claims

1. A light and portable gastrointestinal modular system to remotely and in real time quantify, bioaccesibility or quantity/concentration of metals under conditions of human digestion, metal and metalloids selected from zinc (Zn), lead (Pb), copper (Cu), iron (Fe), nickel (Ni); arsenic (As) and cadmium (Cd), contaminated soil samples, including tailing or mining residues samples, comprising:
a) at least one upper pre-mixing or pre-gastric module to homogenize and conditioning temperature mixture to be analyzed and comprising gastric and enzymatic fluids selected from saliva (S), gastric fluid (G), bile (B) and duodenal fluid (D) and such soil sample, comprising:
a.1) at least one first mixing receiving mean to receive such fluids S and G and such soil sample wherein such first mixing receiving means is of an inert material, including stainless steel or glass,
a.2) at least one first stirring mean, at least one stirring rate sensor, at least one stirring rate regulating mean and at least one first stirring rate controlling mean, located into such at least one first sample receiving means, allowing to keep stirring rate of such at least one first stirring mean allowing to achieve 90% homogeneity,
a.3) at least one first temperature sensor, at least one temperature regulating mean and at least a first temperature controlling mean, located inside such at least one first sample receiving mean allowing to keep temperature inside such at least one first mixture receiving means at a ranging from 35 to 39ºC,
a.4) at least one first pH sensor mean, at least one first pH regulating mean, and at least one first pH controlling mean, located inside such at least first one sample receiving mean, allowing:
a.4.1) regulating pH value at a ranging from 6.0 to 7.0 when such first vessel initially isolately receives such fluid S; or
a.4.2) regulating pH value at a ranging from 1.0 to 1.2 when such first vessel initially isolately receives such fluid G
a.4.3) regulating pH value at a ranging from 1.15 to 1.25, preferably at a pH value 1.2, when such first vessel to receive such soil mixture plus fluid S and fluid G,
a.5) at least one second mixture receiving mean to receive such fluids B and D, and operating in parallel to such at least one mixing receiving mean, and wherein such first mixing receiving mean is of an inert material, including stainless steel or glass;
a.6) at least one second pH sensor mean, at least one second pH regulator mean, and at least one second pH controlling mean located inside such at least second sample receiving mean, allowing:
a.6.1) regulating pH value at a ranging from 7.8 to 8.2 when such first vessel initially isolately receives such fluid B; or
a.6.2) regulating pH value at a ranging from 7.2 to 7.6 when such first vessel initially isolately receives such fluid D
a.7) at least one second temperature sensor, at least one second temperature regulator and at least one second temperature controlling mean, located inside such at least one second sample receptor mean, allowing to keep temperature inside such second mixing receiving mean at a ranging 35 to 39ºC,
a.8) at least one second stirring mean, at least one second stirring rate sensor mean, at least one second stirring rate regulating mean and at least one second stirring rate controlling mean, located inside such at least one second sample receiving mean, allowing to keep stirring rate of such second stirring mean to a stirring rate allowing to achieve 90% homogeneity, and
a.9) at least one screening display interface showing information of such at least pH sensors and such at least first and second stirring rate sensor, and
wherein such at least one first and second temperature controlling means allowing to manually or automatized, in such fist and second temperature regulating means to keep temperature inside such at least first and second mixing receiving means, at 37ºC,
such said at least one first and second stirring means selected from at least one magnetic stirring means,
wherein such at least first and second temperature regulating means are selected from two thermo fans having asynchronous activation;
wherein such magnetic means of such first and second sample receiving mean of pre-mixing module is selected from a ferromagnetic stirring mean without blades,
wherein such first and second sample receiving means are of glass, and
b) at least one intermediate mixing or gastric or gastrointestinal simulator module, comprising:
b.1) at least one bioreactor, preferably stainless steel, receiving such mixture of such soil sample plus fluid S plus fluid G or mixture of such soil sample plus fluid S plus fluid G plus fluid B plus fluid D,
b.2) at least one third stirring mean selected from an "end-over- end"-type stirring allowing to rotate 360º located inside such at least one bioreactor, comprising at least one third stirring rate sensor, at least one a third stirring rate regulator mean and at least a third stepper-type stirring rate controlling mean, allowing to keep stirring rate of such at least third stirring mean allowing to achieve over 50% humidity and at least a third temperature sensor, at least one third temperature regulator mean and at least third temperature controlling means,
wherein such at least one third temperature sensor is selected from an infrared sensor, and
wherein such temperature controlling means of such bioreactor are selected from pneumatic temperature controlling means,
b.3) at least one first base supporting mean to each axis of rotation of such at least one third stirring means, and at least one gear mean mounted over lateral support bearing means, allowing to keep reduced friction with internal wall of such at least one bioreactor, achieving mechanical balance, giving support, having low movement resistance, and moving such at least bioreactor, and at least a third rotation rate sensor mean to such at least third stirring mean, at least a third rotation rate regulating mean to such at least one third stirring mean and at least third rotating rate controlling mean to such at least one third stirring mean,
b.4) at least one third temperature sensor mean, at least one third temperature regulating mean and at least one third temperature controlling mean, located in such at least one bioreactor allowing to keep temperature inside such at least one bioreactor at a ranging from 35 to 39ºC,
b.5) at least one first pH sensor mean, at least one first pH regulating mean, and at least one pH controlling mean allowing to adjusting pH of the contained mixture inside such at least one bioreactor either,
b.5.1) a pH value ranging from 1.15 to 1.25 if mixture entering to such bioreactor corresponds to such mixture of such soil mixture plus fluid S plus fluid G, and a pH value ranging 5.8 to 6.8 if mixture entering to such bioreactor corresponds to such mixture of such soil sample plus fluid S plus fluid G plus fluid B plus fluid D,
b.5.2) a pH value equal or low 1.5 if mixture leaving such bioreactor corresponds to such mixture of such soil sample plus fluid S plus fluid G and at a pH value ranging from 5 to 8 if mixture leaving such bioreactor corresponds to such mixture of such soil sample plus fluid S plus fluid G plus fluid B plus fluid D,
b.6) at least one centrifugating mean allowing to centrifugate mixture of sample with such fluids S, B, G and D contained in such at least one bioreactor,
b.7) at least one metal or metalloid quantifying equipment selected from copper (Cu), lead (Pb), Zinc (Zn) and Iron (Fe), Nickel (Ni); Arsenic (As) and Cadmium (Cd), selected from one of: b.7.1) a photometer, a domestic microwave and a Parr pump,
b.7.2) a mobile quantification mean selected from a ray-X or XRF fluorescence gun, or
b.7.3) heavy metal quantification mean selected from plasma mass spectroscopy (ICP) or atomic absorption;
wherein such at least one third temperature regulating mean is selected from a combination of at least one span and at least one electric resistance;
wherein such at least one third temperature sensor mean measuring temperature inside such at least one third bioreactor and its vicinity, wherein such third stirring rate regulating mean in such at least one bioreactor is selected from a stepper motor,
c) at least one lower recirculation module comprising:
c.1) at least one third receiving mean to receive resulting biochemical extract from mixing or intermediate module by emptying or gravity discharge,
c.2) at least one impeller mean, including a peristaltic pump allowing to recirculate biochemical extract forward the pre-mixing or upper module, and
d) at least one monitoring and data processing mean: stirring rate, in each one of such at least one first, second and third stirring mean; temperature, in each one of such at least one first, second and third receiving means and bioreactor; and pH in each one of such at least one first, second and third receiving means and bioreactor, comprising at least one data center remotely receiving such stirring rate data, temperature and pH from each one of such at least one first, second and third stirring rate sensor means, temperature sensor means and pH sensor mean of such pre-mixing module and intermediate module, wherein such at least one data center comprising at least a database wherein data received is stored from such at least one first, second and third stirring rate sensor mean, temperature sensor and pH sensor, and at least one processor processing such data and sending at least one output signal in response to such at least first, second and third stirring rate controlling mean, temperature controlling mean and pH controlling mean, after compared such data received against stored, referential and pre-set data, enabling a manual or automatized actuation, in one or more of such at least one first, second and third stirring rate controlling means, pH controlling means or temperature controlling mean when a difference is confirmed between such received data and such stored, referential or pre-set data.

2. The system of claim 1 wherein such al least one first and second sample receiving means of such pre-mix module having gates located at the upper part to feeding/entering such fluids S and G.

3. The system of claim 1 wherein such temperature regulating means inside such bioreactor comprising a fan and an electric resistance.

4. The system of claim 1 wherein such first and second sample receiving mean is of glass.

5. The system of claim 1 further comprising means emitting auditive, sonorous signals or both warning to a user that enough time has passed up to achieve 50% homogeneity inside intermediate module.

6. The system of claim 1 wherein such means emitting auditive, sonorous signals or both warning to a user that enough time has passed up to achieve 90% homogeneity.

7. The system of claim 1 wherein such auditive, sonorous signal or both warning to user that preset thermal levels have been exceed.

8. The system of claim 1 wherein such quantifying equipment comprising a photometer, a domestic microwave, a Parr pump and further a thermoreactor.

9. Method for remotely and in real time quantifying, metal and metalloid bioaccesibility in a contaminated soil sample:
a) preparing organic and enzymatic fluids selected from saliva (S), bile (B), gastric fluid (G) and duodenal fluid (D), as the following steps:
a.1) preparing such organic fluid S, adding water including ultra pure water, under stirring up to achieve 90% homogenization and to keep temperature at 28ºC, at a volume/volume rate 3/10, and adding an oxidant agent to regulate pH value at a ranging from 6.0 to 7.0, if necessary, and then gradually heating mixture up to achieve 37ºC wherein such oxidant agent is NaOH;
a.2) preparing such organic fluid G, adding water under stirring up to achieve 90% homogeneity and to keep temperature at 28ºC, in a volume/volume ratio 37/80 and adding an acid reducing agent to regulate pH value at a ranging from 1.0 to 1.2, if necessary, and then gradually heating mixture up to achieve 37ºC;
a.3) preparing such organic fluid B, adding water including ultra pure water stirring up to achieve 90% homogeneity and to keep a temperature of 28ºC, in a volume/volume ratio 3/10, and adding an acid reducing agent to regulate pH value at a ranging from 7.8 to 8.2, if necessary, and then gradually heating mixture up to achieve 37ºC;
a.4) preparing such organic fluid D, adding water, preferably ultra pure water under stirring up to achieve 90% homogeneity and to keep a temperature of 28ºC, in a volume/volume ratio of 37/60, and adding an acid reducing agent to regulate pH value at a ranging from 7.2 to 7,6, if necessary, and then gradually heating mixture up to achieve 37ºC,
wherein such acid regulating agent of any one of steps a.2 to a.4) is hydrochloric acid;
b) preparing a contaminated soil sample drying at a temperature of 40ºC up to achieve 0% humidity and screening up to a size lower 250 micrometers,
c) mixing such resulting soil sample of step b) with such resulting fluid S of step a.1) and such resulting fluid G resulting of step a.2) and let reacting inside bioreactor for a while, and then the resulting mixture is added to react inside reactor with such fluid B resulting of step a.3) and such fluid D resulting of step a.4) to keep temperature of such bioreactor at a ranging from 37 to 39ºC, preferably 37°C, and where such mixtures are performing under stirring up to obtaining over 50% homogeneity, preferably 90%, by the following substeps:
c.1) mixing such soil contaminated sample prepared as step b) having fluid S prepared as step a.1) at a ratio weight/volume of such soil sample to such fluid S (mg/ml) from 100 to 15, under stirring
c.2) adding to such resulting mixture of step c.1), fluid G prepared as step a.2), in a ratio volume to volume S: G of 2: 3, under stirring and regulating pH at a value ranging from 1.15 to 1.25, preferably, at a pH value of 1.2, and let reacting for at least 1 hour,
c.3) adding to such mixture resulting of step c.2), fluid D prepared as step a.3), at a ratio volume to volume S: G: D of 2: 3: 6, under stirring;
c.4) adding to such mixture resulting of step c.3), fluid B prepared as step a.4), at a ratio volume to volume S: G: D: B of 2: 3: 6: 2, under stirring and regulating pH value at a ranging from 5.8 to 6.8, preferably, at a pH value of 6.3, and let reacting for at least 4 hours,
d) centrifugating mixtures resulting of gastric step or step c.2) or resulting of the gastrointestinal step or step c.4) adding an oxidant agent and preparing dilutions with mixtures ranging from 1:200 to 1:10, and one from:
e) eliminating organic material from such dilutions of step d) submitting dilution at:
e.1) an acid organic media to acid digestion,
e.2) a disintegration digestion to metal at:
e.2.1) 120°C in a thermoreactor (method crack set Merck), or
e.2.2) by acid digestion by laboratory microwave (milstone-type),
wherein such pH value is regulated at a range from 6 to 7 prior to quantify one from Fe, Pb, Zn, Cu, Ni, Cd or As, in a photometer,
f) quantifying by separate such metal or metalloid selected from (Cu), iron (Fe), Zinc (Zn) and lead (Pb), Nickel (Ni), Cadmium (Cd) y Arsenic (As), from such samples resulting from step e), in:
f.1) a photometer, domestic microwave and Parr pump,
f.2) a mobile quantification mean selected from a ray-X or XRF fluorescence gun, or
f.3) a heavy metal quantification mean selected from plasma mass spectroscopy (ICP) or atomic absorption,
wherein pH value is regulated prior to quantify such metal in a photometer as step f.1).

10. The method of claim 9 wherein such contaminated soil is selected from tailing soil abandoned at city.

11. The method of claim 9 wherein such contaminated soil is selected from tailing mining soil.

12. The method of claim 9 wherein steps a) and c) are repeated as much times as considered necessary, wherein fluids S, B, D, G prepared are mixed with the mixture resulting of step c) before each repeating cycle.

13. The method of claim 9 further comprising to emit visual, sonorous signals or both to warning to a user that enough time has passed up to achieving over 50% mixture homogeneity either when such soil sample is mixed with S and G or when such sample is mixed with S, G, B and D, and wherein such enough time is 1 hour to such mixture of such soil sample with S and G.

14. The method of claim 9 wherein such visual, sonorous signal or both warning to a user that enough time has passed up to achieve 90% homogeneity and such enough time is 4 hours to such mixture of such soil sample with S, G, B and D.

15. The method of claim 9 wherein such pH value is regulated with NaOH and HCl prior to quantifying Fe.

16. The method of claim 9 wherein such pH value is regulated with diluted NH₃ and diluted HNO₃ prior to quantify Pb.

17. The method of claim 9 wherein such pH value is regulated with NaOH and H₂SO₄ prior to quantify one from Zn, Cu, Ni, Cd o As.

18. The method of claim 9 wherein such metal disintegration digestion of step e) is carried out in a thermoreactor with reagents added as such metal desired to be quantified and as manufacturer instructions.

19. The method of claim 9 wherein such pH value is regulated at a ranging from 3 to 6 prior to quantify Pb, at step f.1).

20. The method of claim 9 wherein such pH value is regulated at a ranging from de 1 to 10 to quantify Fe, at step f.1).

21. The method of claim 9 wherein such pH value is regulated at a ranging from 4 to 10 to quantify Zn, at step f.1).

22. The method of claim 9 wherein such pH value is regulated at a ranging from 3 to 10 to quantify Cu, at step f.1).

23. The method of claim 9 wherein such pH value is regulated at a ranging from 3 to 8 prior to quantify Ni, at step f.1).

## Patentansprüche

1. Leichtes und tragbares gastrointestinales modulares System zur Quantifizierung der Biozugänglichkeit oder Anzahl/Konzentration von Metallen unter Bedingungen der humanen Verdauung, wobei die Metalle und Metalloide aus Zink (Zn), Blei (Pb), Kupfer (Cu), Eisen (Fe), Nickel (Ni), Arsen (As) und Cadmium (Cd) ausgewählt werden, in Echtzeit und aus der Ferne, in Proben von kontaminiertem Boden, einschließlich Proben von Abraum oder Bergbaurückständen, umfassend:
a) mindestens ein oberes vormischendes oder vorgastrisches Modul zum Homogenisieren und Konditionieren des zu analysierenden Temperaturgemischs und umfassend gastrische und enzymatische Fluide, ausgewählt aus Speichel (S), gastrischem Fluid (G), Galle (B) und duodenalem Fluid (D) und eine solche Bodenprobe, umfassend:
a.1) mindestens ein erstes Mischaufnahmemittel, um solche Fluide S und G und solche Bodenprobe aufzunehmen, wobei das erste Mischaufnahmemittel aus einem inerten Material besteht, einschließlich Edelstahl oder Glas,
a.2) mindestens ein erstes Rührmittel, mindestens einen Rührgeschwindigkeitssensor, mindestens ein Rührgeschwindigkeitsregelungsmittel und mindestens ein erstes Rührgeschwindigkeitssteuerungsmittel, die sich in einem solchem mindestens einem ersten Probeaufnahmemittel befinden, wobei die Rührgeschwindigkeit eines solchen mindestens einen ersten Rührmittels aufrechterhalten werden kann, wodurch eine Homogenität von 90 % erreicht werden kann,
a.3) mindestens einen ersten Temperatursensor, mindestens ein Temperaturregelungsmittel und mindestens ein erstes Temperatursteuerungsmittel, die sich in einem solchen mindestens einem ersten Probenaufnahmemittel befinden, wobei die Temperatur in einem solchen mindestens einen ersten Gemischaufnahmemittel in einem Bereich von 35 bis 39 °C aufrechterhalten werden kann,
a.4) mindestens ein erstes pH-Sensormittel, mindestens ein erstes pH-Regelungsmittel und mindestens ein erstes pH-Steuerungsmittel, die sich in einem solchen mindestens einem ersten Probeaufnahmemittel befinden, wobei Folgendes ermöglicht wird:
a.4.1) Regelung des pH-Wertes in einem Bereich von 6,0 bis 7,0, wenn ein solches erstes Gefäß ein solches Fluid S anfänglich isoliert aufnimmt; oder a.4.2) Regelung des pH-Wertes in einem Bereich von 1,0 bis 1,2, wenn ein solches erstes Gefäß ein solches Fluid G anfänglich isoliert aufnimmt a.4.3) Regelung des pH-Werts in einem Bereich von 1,15 bis 1,25, vorzugsweise bei einem pH-Wert von 1,2, wenn ein solches erstes Gefäß eine solche Bodenmischung plus Fluid S und Fluid G aufnimmt,
a.5) mindestens ein zweites Gemischaufnahmemittel zum Aufnehmen solcher Fluide B und D, und das parallel zu einem solchen mindestens einen Mischaufnahmemittel arbeitet, und wobei ein solches erstes Mischaufnahmemittel aus einem inerten Material besteht, einschließlich Edelstahl oder Glas;
a.6) mindestens ein zweites pH-Sensormittel, mindestens ein zweites pH-Regler-Mittel und mindestens ein zweites pH-Steuerungsmittel, das sich in einem solchen mindestens zweiten Probeaufnahmemittel befindet, wobei Folgendes ermöglicht wird:
a.6.1) Regelung des pH-Wertes in einem Bereich von 7,8 bis 8,2, wenn ein solches erstes Gefäß ein solches Fluid B anfänglich isoliert aufnimmt; oder a.6.2) Regelung des pH-Wertes in einem Bereich von 7,2 bis 7,6, wenn ein solches erstes Gefäß ein solches Fluid D anfänglich isoliert aufnimmt a.7) mindestens einen zweiten Temperatursensor, mindestens einen zweiten Temperaturregler und mindestens ein zweites Temperatursteuerungsmittel, die sich in einem solchen mindestens einen zweiten Probeaufnehmermittel befinden, wobei die Temperatur in einem solchen zweiten Mischaufnahmemittel in einem Bereich von 35 bis 39 °C aufrechterhalten werden kann,
a.8) mindestens ein zweites Rührmittel, mindestens ein zweites Rührgeschwindigkeitssensormittel, mindestens ein zweites Rührgeschwindigkeitsregelungsmittel und mindestens ein zweites Rührgeschwindigkeitssteuerungsmittel, die sich in einem solchen mindestens einen zweiten Probeaufnahmemittel befinden, wobei die Rührgeschwindigkeit eines solchen zweiten Rührmittels auf einer Rührgeschwindigkeit aufrechterhalten werden kann, wodurch eine Homogenität von 90 % erreicht werden kann, und
a.9) mindestens eine Bildschirm-Benutzeroberfläche, die Informationen von solchen mindestens pH-Sensoren und solchen mindestens ersten und zweiten Rührgeschwindigkeitssensor zeigt, und
wobei ein solches mindestens erstes und zweites Temperatursteuerungsmittel ermöglicht, manuell oder automatisiert in einem solchen ersten und zweiten Temperaturregelungsmittel die Temperatur in einem solchen mindestens ersten und zweiten Mischaufnahmemittel bei 37 °C aufrechtzuerhalten,
wobei solches mindestens ein erstes und zweites Rührmittel aus mindestens einem magnetischen Rührmittel ausgewählt wird,
wobei solche mindestens ein erstes und ein zweites Temperaturregelungsmittel aus zwei Thermogebläsen ausgewählt sind, die eine asynchrone Aktivierung aufweisen;
wobei solche magnetischen Mittel solcher erster und zweiter Probeaufnahmemittel des vormischenden Moduls aus einem ferromagnetischen Rührmittel ohne Schaufeln ausgewählt sind,
wobei solche ersten und zweiten Probeaufnahmemittel aus Glas bestehen, und
b) mindestens ein dazwischenliegendes vermischendes oder gastrisches oder gastrointestinales Simulatormodul, umfassend:
b.1) mindestens einen Bioreaktor, vorzugsweise aus rostfreiem Stahl, zum Aufnehmen eines solchen Gemischs einer solchen Bodenprobe plus Fluid S plus Fluid G oder eines Gemischs aus einer solchen Bodenprobe plus Fluid S plus Fluid G plus Fluid B plus Fluid D,
b.2) mindestens ein drittes Rührmittel, ausgewählt aus einem Rührmittel vom Typ "End-über-End", das eine Drehung um 360° ermöglicht und sich innerhalb eines solchen mindestens einen Bioreaktors befindet, umfassend mindestens einen dritten Rührgeschwindigkeitssensor, mindestens ein drittes Rührgeschwindigkeitsregelungsmittel und mindestens ein drittes Rührgeschwindigkeitssteuerungsmittel vom Schrittmotortyp, wobei die Rührgeschwindigkeit des solchen mindestens dritten Rührmittels aufrechterhalten werden kann, wodurch eine Luftfeuchtigkeit von über 50 % erreicht werden kann, und mindestens einen dritten Temperatursensor, mindestens ein drittes Temperaturregelungsmittel und mindestens ein drittes Temperatursteuerungsmittel,
wobei ein solcher mindestens ein dritter Temperatursensor aus einem Infrarotsensor ausgewählt ist und
wobei solche Temperatursteuerungsmittel eines solchen Bioreaktors aus pneumatischen Temperatursteuerungsmitteln ausgewählt sind,
b.3) mindestens ein erstes Basisträgermittel für jede Drehachse von einem solchen mindestens einem dritten Rührmittel und mindestens ein Getriebemittel, das über seitliche Trägerlagermittel montiert ist, wobei die Reibung mit der Innenwand von einem solchen mindestens einem Bioreaktor reduziert werden kann, wodurch ein mechanisches Gleichgewicht erreicht wird, das Unterstützung bietet, einen geringen Bewegungswiderstand aufweist und einen solchen mindestens einen Bioreaktor bewegt, und mindestens ein drittes Rotationsgeschwindigkeitssensormittel für ein solches mindestens drittes Rührmittel, mindestens ein drittes Rotationsgeschwindigkeitsregelungsmittel für ein solches mindestens ein drittes Rührmittel und mindestens ein drittes Rotationsgeschwindigkeitssteuerungsmittel für ein solches mindestens ein drittes Rührmittel,
b.4) mindestens ein drittes Temperatursensormittel, mindestens ein drittes Temperaturregelungsmittel und mindestens ein drittes Temperatursteuerungsmittel, die sich in einem solchen mindestens einen Bioreaktor befinden, wobei die Temperatur in einem solchen mindestens einen Bioreaktor in einem Bereich von 35 bis 39 °C aufrechterhalten werden kann,
b.5) mindestens ein erstes pH-Sensormittel, mindestens ein erstes pH-Regelungsmittel und mindestens ein pH-Steuerungsmittel, wobei das Anpassen des pH-Werts des enthaltenen Gemischs in einem solchen mindestens einen Bioreaktor ermöglicht wird, entweder
b.5.1) einen pH-Wert im Bereich von 1,15 bis 1,25, falls das in einen solchen Bioreaktor eintretende Gemisch einem solchen Gemisch aus einer solchen Bodenmischung plus Fluid S plus Fluid G entspricht, und einen pH-Wert im Bereich von 5,8 bis 6,8, falls das in einen solchen Bioreaktor eintretende Gemisch einem solchen Gemisch aus einer solchen Bodenprobe plus Fluid S plus Fluid G plus Fluid B plus Fluid D entspricht,
b.5.2) einen pH-Wert von gleich oder weniger als 1,5, falls das Gemisch, das aus einem solchen Bioreaktor austritt, einem solchen Gemisch aus einer solchen Bodenprobe plus Fluid S plus Fluid G entspricht, und einen pH-Wert im Bereich von 5 bis 8, falls das Gemisch, das aus einem solchen Bioreaktor austritt, einem solchen Gemisch aus einer solchen Bodenprobe plus Fluid S plus Fluid G plus Fluid B plus Fluid D entspricht,
b.6) mindestens ein Zentrifugationsmittel, wobei das Gemisch der Probe mit solchen Fluiden S, B, G und D, die in einem solchen mindestens einen Bioreaktor enthalten sind, zentrifugiert werden kann,
b.7) mindestens eine Einrichtung zur Quantifizierung von Metallen oder Metalloiden, die aus Kupfer (Cu), Blei (Pb), Zink (Zn) und Eisen (Fe), Nickel (Ni); Arsen (As) und Cadmium (Cd) ausgewählt sind, ausgewählt aus einem von:
b.7.1) ein Photometer, eine Haushaltsmikrowelle und eine Parr-Pumpe,
b.7.2) ein mobiles Quantifizierungsmittel, ausgewählt aus einer Röntgenfluoreszenz- oder XRF-Fluoreszenzkanone, oder
b.7.3) Schwermetallquantifizierungsm ittel, ausgewählt aus Plasmamassenspektroskopie (ICP) oder Atomabsorption;
wobei solches mindestens ein drittes Temperaturregelungsmittel aus einer Kombination von mindestens einem Gespann und mindestens einem elektrischen Widerstand ausgewählt wird;
wobei ein solcher mindestens ein dritter Temperatursensormittel die Temperatur in einem solchen mindestens einen dritten Bioreaktor und seiner Umgebung misst, wobei ein solches drittes Rührgeschwindigkeitsregelungsmittel in einem solchen mindestens einen Bioreaktor aus einem Schrittmotor ausgewählt ist,
c) mindestens ein unteres Rezirkulationsmodul, umfassend:
c.1) mindestens ein drittes Aufnahmemittel zum Aufnehmen des resultierenden biochemischen Extrakts aus dem vermischenden oder dazwischenliegenden Modul durch Entleeren oder Schwerkraftentladung,
c.2) mindestens ein Vorantreibmittel, einschließlich einer peristaltischen Pumpe, wodurch das Rezirkulieren des biochemischen Extrakts vor dem vormischenden oder oberen Modul ermöglicht wird, und
d) mindestens ein Überwachungs- und Datenverarbeitungsmittel: Rührgeschwindigkeit, in jedem von solchen mindestens einem ersten, zweiten und dritten Rührmittel; Temperatur, in jedem von solchen mindestens einem ersten, zweiten und dritten Aufnahmemittel und Bioreaktor; und pH-Wert, in jedem von solchen mindestens einem ersten, zweiten und dritten Aufnahmemittel und Bioreaktor, umfassend mindestens ein Rechenzentrum, das solche Rührgeschwindigkeits-, Temperatur- und pH-Daten von jedem von solchen mindestens einem ersten, zweiten und dritten Rührgeschwindigkeitssensormittel, Temperatursensormittel und pH-Sensormittel eines solchen vorvermischenden Moduls und dazwischenliegenden Moduls aus der Ferne empfängt, wobei ein solches mindestens ein Rechenzentrum mindestens eine Datenbank umfasst, wobei Daten, die von solchen mindestens einem ersten, zweiten und dritten Rührgeschwindigkeitssensormittel, Temperatursensor und pH-Sensor, gespeichert werden, und mindestens einen Prozessor, der diese Daten verarbeitet und mindestens ein Ausgangssignal als Reaktion auf ein solches mindestens erstes, zweites und drittes Rührgeschwindigkeitssteuerungsmittel, Temperatursteuerungsmittel und pH-Steuerungsmittel sendet, nach dem Vergleich solch empfangener Daten mit gespeicherten, referenziellen und voreingestellten Daten, wobei eine manuelle oder automatisierte Betätigung in einem oder mehreren von solchen mindestens einem ersten, zweiten und dritten Rührgeschwindigkeitssteuerungsmittel, pH-Steuerungsmittel oder Temperatursteuerungsmittel ermöglicht wird, wenn eine Differenz zwischen solchen empfangenen Daten und solchen gespeicherten, referenziellen oder voreingestellten Daten bestätigt wird.

2. System nach Anspruch 1, wobei ein solches mindestens ein erstes und zweites Probeaufnahmemittel eines solchen vormischenden Moduls Schleusen aufweist, die sich am oberen Teil zur Einspeisung/Eingabe solcher Fluide S und G befinden.

3. System nach Anspruch 1, wobei ein solches Temperaturregelungsmittel in einem solchen Bioreaktor ein Gebläse und einen elektrischen Widerstand umfasst.

4. System nach Anspruch 1, wobei ein solches erstes und zweites Probenaufnahmemittel aus Glas besteht.

5. System nach Anspruch 1, ferner umfassend Mittel, die akustische, klangliche Signale oder beides emittieren, um einen Benutzer zu warnen, dass genügend Zeit verstrichen ist, um 50 % Homogenität in dem dazwischenliegenden Modul zu erreichen.

6. System nach Anspruch 1, wobei solche Mittel akustische, klangliche Signale oder beides emittieren, um einen Benutzer zu warnen, dass genügend Zeit verstrichen ist, um 90 % Homogenität zu erreichen.

7. System nach Anspruch 1, wobei ein solches auditives, klangliches Signal oder beide den Benutzer warnen, dass voreingestellte thermische Niveaus überschritten wurden.

8. System nach Anspruch 1, wobei eine solche Einrichtung zur Quantifizierung ein Photometer, eine Haushaltsmikrowelle, eine Parr-Pumpe und ferner einen Thermoreaktor umfasst.

9. Verfahren zur Quantifizierung der Biozugänglichkeit von Metallen und Metalloiden in einer kontaminierten Bodenprobe:
a) Herstellen von organischen und enzymatischen Fluiden, die aus Speichel (S), Galle (B), gastrischem Fluid (G) und duodenalem Fluid (D) ausgewählt werden, in den folgenden Schritten:
a.1) Herstellen eines solchen organischen Fluids S, Hinzufügen von Wasser, einschließlich ultrareinem Wasser, unter Rühren, um eine 90%ige Homogenisierung zu erreichen und die Temperatur bei 28 °C aufrechtzuerhalten, bei einem Volumen/Volumen-Verhältnis von 3/10, und Hinzufügen eines oxidierenden Mittels, um den pH-Wert bei Bedarf in einem Bereich von 6,0 bis 7,0 zu regulieren, und dann allmähliches Erwärmen des Gemischs, um 37 °C zu erreichen, wobei das oxidierende Mittel NaOH ist;
a.2) Herstellen eines solchen organischen Fluids G, Hinzufügen von Wasser unter Rühren, um eine Homogenität von 90 % zu erreichen und die Temperatur bei 28 °C aufrechtzuerhalten, in einem Volumen/Volumen-Verhältnis von 37/80 und Hinzufügen eines säurereduzierenden Mittels, um den pH-Wert bei Bedarf in einem Bereich von 1,0 bis 1,2 zu regulieren, und dann allmähliches Erwärmen des Gemischs, um 37 °C zu erreichen;
a.3) Herstellen eines solchen organischen Fluids B, Hinzufügen von Wasser, einschließlich ultrareinem Wasser, unter Rühren, um eine Homogenität von 90 % zu erreichen und die Temperatur bei 28 °C aufrechtzuerhalten, bei einem Volumen/Volumen-Verhältnis von 3/10, und Hinzufügen eines säurereduzierenden Mittels, um den pH-Wert bei Bedarf in einem Bereich von 7,8 bis 8,2 zu regulieren, und dann allmähliches Erwärmen des Gemischs, um 37 °C zu erreichen;
a.4) Herstellen eines solchen organischen Fluids D, Hinzufügen von Wasser, vorzugsweise von ultrareinem Wasser unter Rühren, um eine Homogenität von 90 % zu erreichen und eine Temperatur von 28 °C aufrechtzuerhalten, in einem Volumen/Volumen-Verhältnis von 37/60 und Hinzufügen eines säurereduzierenden Mittels, um den pH-Wert bei Bedarf in einem Bereich von 7,2 bis 7,6 zu regulieren, und dann allmähliches Erwärmen des Gemischs, um 37 °C zu erreichen,
wobei ein solches säureregulierende Mittel in einem der Schritte a.2 bis a.4) Salzsäure ist;
b) Vorbereiten einer Probe des verunreinigten Bodens durch Trocknen bei einer Temperatur von 40 °C, um eine Feuchtigkeit von 0 % zu erreichen, und Sieben bis zu einer Größe von weniger als 250 Mikrometern,
c) Mischen einer solchen resultierenden Bodenprobe aus Schritt b) mit einem solchen resultierenden Fluid S aus Schritt a.1) und einem solchen resultierenden Fluid G aus Schritt a.2) und eine Weile im Bioreaktor reagieren lassen, und dann wird das resultierende Gemisch hinzugefügt, um im Reaktor mit einem solchen aus Schritt a.3) resultierenden Fluid B und einem solchen aus Schritt a.4) resultierenden Fluid D zu reagieren, um die Temperatur eines solchen Bioreaktors in einem Bereich von 37 bis 39 °C, vorzugsweise 37 °C, aufrechtzuerhalten, und wobei solche Gemische unter Rühren durchgeführt werden, um eine Homogenität von über 50 %, vorzugsweise 90 %, zu erlangen, durch die folgenden Unterschritte:
c.1) Mischen einer solchen kontaminierten Bodenprobe, vorbereitet in Schritt b), die Fluid S, vorbereitet in Schritt a.1), aufweist, in einem Verhältnis von Gewicht/Volumen einer solchen Bodenprobe zu einem solchen Fluid S (mg/ml) von 100 bis 15, unter Rühren
c.2) Hinzufügen von Fluid G, vorbereitet in Schritt a.2), zu einem solchen resultierenden Gemisch von Schritt c.1) in einem Verhältnis von Volumen zu Volumen S:G von 2:3 unter Rühren und Regulieren des pH-Werts auf einen Wert im Bereich von 1,15 bis 1,25, vorzugsweise auf einen pH-Wert von 1,2, und Reagierenlassen für mindestens 1 Stunde,
c.3) Hinzufügen von Fluid D, vorbereitet in Schritt a.3), zu einem solchen aus Schritt c.2) resultierenden Gemisch unter Rühren in einem Verhältnis von Volumen zu Volumen S:G:D von 2:3:6;
c.4) Hinzufügen von Fluid B, vorbereitet in Schritt a.4), zu einem solchen aus Schritt c.3) resultierenden Gemisch in einem Verhältnis von Volumen zu Volumen S:G:D:B von 2:3:6:2, unter Rühren und Regulieren des pH-Werts in einem Bereich von 5,8 bis 6,8, vorzugsweise auf einen pH-Wert von 6,3, und Reagierenlassen für mindestens 4 Stunden,
d) Zentrifugieren von Gemischen, die aus dem gastrischen Schritt oder Schritt c.2) resultieren oder aus dem gastrointestinalen Schritt oder Schritt c.4) resultieren, Hinzufügen eines oxidierenden Mittels und Herstellen von Verdünnungen mit Gemischen im Bereich von 1:200 bis 1:10, und eines von:
e) Entfernen von organischem Material aus solchen Verdünnungen von Schritt d), Aussetzen der Verdünnung zu Folgendem:
e.1) einem sauren organischen Medium zum Säureaufschluss,
e.2) einem Zersetzungsaufschluss zu Metall bei:
e.2.1) 120 °C in einem Thermoreaktor (Crack- Set-Verfahren von Merck) oder
e.2.2) durch Säureaufschluss mittels Labormikrowelle (Milstone-Typ),
wobei ein solcher pH-Wert auf einen Bereich von 6 bis 7 geregelt wird, bevor eines von Fe, Pb, Zn, Cu, Ni, Cd oder As in einem Photometer quantifiziert wird,
f) Quantifizieren durch Trennen solcher Metalle oder Metalloide, die aus (Cu), Eisen (Fe), Zink (Zn) und Blei (Pb), Nickel (Ni), Cadmium (Cd) und Arsen (As) ausgewählt werden, aus solchen Proben, die aus Schritt e) resultieren, in:
f.1) einem Photometer, einer Haushaltsmikrowelle und einer Parr-Pumpe,
f.2) einem mobilen Quantifizierungsmittel, ausgewählt aus einer Röntgenfluoreszenz- oder XRF-Fluoreszenzkanone, oder
f.3) einem Schwermetall-Quantifizierungsmittel, ausgewählt aus Plasmamassenspektroskopie (ICP) oder Atomabsorption,
wobei der pH-Wert vor dem Quantifizieren eines solchen Metalls in einem Photometer in Schritt f.1) geregelt wird.

10. Verfahren nach Anspruch 9, wobei solch kontaminierter Boden aus in der Stadt aufgegebenem Abraum ausgewählt wird.

11. Verfahren nach Anspruch 9, wobei solch kontaminierter Boden aus Abraum aus dem Bergbau ausgewählt wird.

12. Verfahren nach Anspruch 9, wobei die Schritte a) und c) so oft wiederholt werden, wie dies für erforderlich gehalten wird, wobei die vorbereiteten Fluide S, B, D, G vor jedem Wiederholungszyklus mit dem aus Schritt c) resultierenden Gemisch gemischt werden.

13. Verfahren nach Anspruch 9, ferner umfassend das Emittieren von visuellen, klanglichen Signalen oder beiden, um einen Benutzer zu warnen, dass genügend Zeit verstrichen ist, um eine Gemischhomogenität von über 50 % zu erreichen, entweder wenn eine solche Bodenprobe mit S und G gemischt wird oder wenn eine solche Probe mit S, G, B und D gemischt wird, und wobei eine solche ausreichende Zeit 1 Stunde für ein solches Gemisch einer solchen Bodenprobe mit S und G beträgt.

14. Verfahren nach Anspruch 9, wobei ein solches visuelles, klangliches Signal oder beide den Benutzer warnen, dass genügend Zeit verstrichen ist, um eine Homogenität von 90 % zu erreichen, und diese ausreichende Zeit 4 Stunden für ein solches Gemisch einer solchen Bodenprobe mit S, G, B und D beträgt.

15. Verfahren nach Anspruch 9, wobei ein solcher pH-Wert vor der Quantifizierung von Fe mit NaOH und HCl geregelt wird.

16. Verfahren nach Anspruch 9, wobei ein solcher pH-Wert vor der Quantifizierung von Pb mit verdünntem NH₃ und verdünnter HNOs geregelt wird.

17. Verfahren nach Anspruch 9, wobei ein solcher pH-Wert vor der Quantifizierung von einem aus Zn, Cu, Ni, Cd oder As mit NaOH und H₂SO₄ geregelt wird.

18. Verfahren nach Anspruch 9, wobei ein solcher Zersetzungsaufschluss von Metall von Schritt e) in einem Thermoreaktor durchgeführt wird, wobei Reagenzien hinzugefügt werden, wenn ein solches Metall quantifiziert werden soll, und gemäß den Anweisungen des Herstellers.

19. Verfahren nach Anspruch 9, wobei ein solcher pH-Wert vor der Quantifizierung von Pb in Schritt f.1) in einem Bereich von 3 bis 6 geregelt wird.

20. Verfahren nach Anspruch 9, wobei ein solcher pH-Wert vor der Quantifizierung von Fe in Schritt f.1) in einem Bereich von 1 bis 10 geregelt wird.

21. Verfahren nach Anspruch 9, wobei ein solcher pH-Wert zur Quantifizierung von Zn in Schritt f.1) in einem Bereich von 4 bis 10 geregelt wird.

22. Verfahren nach Anspruch 9, wobei ein solcher pH-Wert zur Quantifizierung von Cu in Schritt f.1) in einem Bereich von 3 bis 10 geregelt wird.

23. Verfahren nach Anspruch 9, wobei ein solcher pH-Wert vor der Quantifizierung von Ni in Schritt f.1) in einem Bereich von 3 bis 8 geregelt wird.

## Revendications

1. Système modulaire gastro-intestinal léger et portatif pour quantifier à distance et en temps réel la bioaccessibilité ou la quantité/concentration de métaux dans des conditions de digestion humaine, les métaux et métalloïdes étant choisis parmi le zinc (Zn), le plomb (Pb), le cuivre (Cu), le fer (Fe), le nickel (Ni) ; l'arsenic (As) et le cadmium (Cd), dans des échantillons de sol contaminé, notamment des échantillons de rejets ou de résidus miniers, comprenant :
a) au moins un module supérieur de pré-mélange ou pré-gastrique pour homogénéiser et conditionner le mélange à température à analyser et comprenant des liquides gastriques et enzymatiques choisis parmi la salive (S), le liquide gastrique (G), la bile (B) et le liquide duodénal (D) et un tel échantillon de sol, comprenant :
a.1) au moins un premier moyen de réception et de mélange pour recevoir de tels liquides S et G et un tel échantillon de sol, un tel premier moyen de réception et de mélange étant constitué d'un matériau inerte, notamment l'acier inoxydable ou le verre,
a.2) au moins un premier moyen d'agitation, au moins un capteur de vitesse d'agitation, au moins un moyen de régulation de vitesse d'agitation et au moins un premier moyen de contrôle de vitesse d'agitation, situés dans un tel au moins un premier moyen de réception d'échantillon, permettant de maintenir la vitesse d'agitation d'un tel au moins un premier moyen d'agitation permettant d'atteindre une homogénéité de 90 %,
a.3) au moins un premier capteur de température, au moins un moyen de régulation de température et au moins un premier moyen de contrôle de température, situés à l'intérieur d'un tel au moins un premier moyen de réception d'échantillon permettant de maintenir la température à l'intérieur d'un tel au moins un premier moyen de réception de mélange dans une plage allant de 35 à 39 °C,
a.4) au moins un premier moyen capteur de pH, au moins un premier moyen de régulation de pH et au moins un premier moyen de contrôle de pH, situés à l'intérieur d'un tel au moins un premier moyen de réception d'échantillon, permettant :
a.4.1) la régulation de la valeur de pH dans une plage allant de 6,0 à 7,0 lorsqu'un tel premier récipient reçoit initialement isolément un tel liquide S ; ou
a.4.2) la régulation de la valeur de pH dans une plage allant de 1,0 à 1,2 lorsqu'un tel premier récipient reçoit initialement isolément un tel liquide G a.4.3) la régulation de la valeur de pH dans une plage allant de 1,15 à 1,25, de préférence à une valeur de pH de 1,2, lorsqu'un tel premier récipient reçoit un tel mélange de sol avec du liquide S et du liquide G,
a.5) au moins un deuxième moyen de réception de mélange pour recevoir de tels liquides B et D et fonctionnant en parallèle avec un tel au moins un moyen de réception et de mélange et un tel premier moyen de réception et de mélange étant constitué d'un matériau inerte, notamment l'acier inoxydable ou le verre ;
a.6) au moins un deuxième moyen capteur de pH, au moins un deuxième moyen régulateur de pH et au moins un deuxième moyen de contrôle de pH situés à l'intérieur d'un tel au moins un deuxième moyen de réception d'échantillon, permettant :
a.6.1) la régulation de la valeur de pH dans une plage allant de 7,8 à 8,2 lorsqu'un tel premier récipient reçoit initialement isolément un tel liquide B ; ou
a.6.2) la régulation de la valeur de pH dans une plage allant de 7,2 à 7,6 lorsqu'un tel premier récipient reçoit initialement isolément un tel liquide D
a.7) au moins un deuxième capteur de température, au moins un deuxième régulateur de température et au moins un deuxième moyen de contrôle de température, situés à l'intérieur d'un tel au moins un deuxième moyen récepteur d'échantillon, permettant de maintenir la température à l'intérieur d'un tel deuxième moyen de réception et de mélange dans une plage allant de 35 à 39 °C,
a.8) au moins un deuxième moyen d'agitation, au moins un deuxième moyen capteur de vitesse d'agitation, au moins un deuxième moyen de régulation de vitesse d'agitation et au moins un deuxième moyen de contrôle de vitesse d'agitation, situés à l'intérieur d'un tel au moins un deuxième moyen de réception d'échantillon, permettant de maintenir la vitesse d'agitation d'un tel deuxième moyen d'agitation à une vitesse d'agitation permettant d'atteindre une homogénéité de 90 % et
a.9) au moins une interface d'affichage à l'écran montrant des informations sur de tels au moins capteurs de pH et de tels au moins un premier et au moins un deuxième capteurs de vitesse d'agitation et
de tels au moins un premier et au moins un deuxième moyens de contrôle de température permettant manuellement ou de façon automatisée de maintenir la température à l'intérieur de tels au moins un premier et au moins un deuxième moyens de réception et de mélange dans de tels premier et deuxième moyens de régulation de température à 37 °C,
de tels dits au moins un premier et au moins un deuxième moyens d'agitation en étant au moins un choisi parmi des moyens d'agitation magnétiques,
de tels au moins un premier et au moins un deuxième moyens de régulation de température étant choisis parmi deux ventilateurs thermiques ayant une activation asynchrone ;
de tels moyens magnétiques de tels premier et deuxième moyens de réception d'échantillon du module de pré-mélange étant choisis parmi des moyens d'agitation ferromagnétiques sans pales,
de tels premier et deuxième moyens de réception d'échantillon étant en verre, et
b) au moins un module simulateur gastrique ou gastro-intestinal ou de mélange intermédiaire, comprenant :
b.1) au moins un bioréacteur, de préférence en acier inoxydable, recevant un tel mélange d'un tel échantillon de sol avec du liquide S et avec du liquide G ou un mélange d'un tel échantillon de sol avec du liquide S, avec du liquide G, avec du liquide B et avec du liquide D,
b.2) au moins un troisième moyen d'agitation choisi parmi des moyens d'agitation de type « à tambour vertical » permettant de tourner sur 360° situé à l'intérieur d'un tel au moins un bioréacteur, comprenant au moins un troisième capteur de vitesse d'agitation, au moins un troisième moyen régulateur de vitesse d'agitation et au moins un troisième moyen de contrôle de vitesse d'agitation de type pas à pas, permettant de maintenir une vitesse d'agitation d'un tel au moins un troisième moyen d'agitation permettant d'atteindre une humidité de plus de 50 %, et au moins un troisième capteur de température, au moins un troisième moyen régulateur de température et au moins un troisième moyen de contrôle de température,
un tel au moins un troisième capteur de température étant choisi parmi des capteurs à infrarouge et
un tel moyen de contrôle de température d'un tel bioréacteur étant choisi parmi des moyens de contrôle de température pneumatiques,
b.3) au moins un premier moyen de support de base pour chaque axe de rotation d'un tel au moins un troisième moyen d'agitation et au moins un moyen à engrenages monté sur un moyen palier de support latéral, permettant de maintenir un frottement réduit avec la paroi interne d'un tel au moins un bioréacteur, atteignant un équilibre mécanique, fournissant un support, ayant une faible résistance au déplacement et déplaçant de tels au moins un bioréacteur et au moins un troisième moyen capteur de vitesse de rotation vers un tel au moins un troisième moyen d'agitation, au moins un troisième moyen de régulation de vitesse de rotation vers un tel au moins un troisième moyen d'agitation et au moins un troisième moyen de contrôle de vitesse de rotation vers un tel au moins un troisième moyen d'agitation,
b.4) au moins un troisième moyen capteur de température, au moins un troisième moyen de régulation de température et au moins un troisième moyen de contrôle de température, situés dans un tel au moins un bioréacteur permettant de maintenir la température à l'intérieur d'un tel au moins un bioréacteur dans une plage allant de 35 à 39 °C,
b.5) au moins un premier moyen capteur de pH, au moins un premier moyen de régulation de pH et au moins un moyen de contrôle de pH permettant d'ajuster le pH du mélange contenu à l'intérieur d'un tel au moins un bioréacteur à soit,
b.5.1) une valeur de pH allant de 1,15 à 1,25 si le mélange entrant dans un tel bioréacteur correspond à un tel mélange d'un tel mélange de sol avec du liquide S et avec du liquide G et une valeur de pH allant de 5,8 à 6,8 si le mélange entrant dans un tel bioréacteur correspond à un tel mélange d'un tel échantillon de sol avec du liquide S, avec du liquide G, avec du liquide B et avec du liquide D,
b.5.2) une valeur de pH inférieure ou égale à 1,5 si le mélange sortant d'un tel bioréacteur correspond à un tel mélange d'un tel échantillon de sol avec du liquide S et avec du liquide G et à une valeur de pH allant de 5 à 8 si le mélange sortant d'un tel bioréacteur correspond à un tel mélange d'un tel échantillon de sol avec du liquide S, avec du liquide G, avec du liquide B et avec du liquide D,
b.6) au moins un moyen de centrifugation permettant de centrifuger le mélange d'échantillon avec de tels liquides S, B, G et D contenus dans un tel au moins un bioréacteur,
b.7) au moins un équipement pour quantifier des métaux ou métalloïdes choisis parmi le cuivre (Cu), le plomb (Pb), le zinc (Zn) et le fer (Fe), le nickel (Ni) ; l'arsenic (As) et le cadmium (Cd), choisi parmi l'un de :
b.7.1) un photomètre, un four à micro-ondes ménager et une pompe Parr,
b.7.2) un moyen de quantification mobile choisi parmi un canon à rayons X ou de fluorescence des rayons X ou
b.7.3) un moyen de quantification de métaux lourds choisi parmi la spectroscopie de masse à plasma (ICP) ou l'absorption atomique ;
un tel au moins un troisième moyen de régulation de température étant choisi parmi des combinaisons d'au moins un intervalle et d'au moins une résistance électrique ;
un tel au moins un troisième moyen capteur de température mesurant la température à l'intérieur d'un tel au moins un troisième bioréacteur et de son voisinage, un tel troisième moyen de régulation de vitesse d'agitation dans un tel au moins un bioréacteur étant choisi parmi des moteurs pas à pas,
c) au moins un module de recirculation inférieur comprenant :
c.1) au moins un troisième moyen de réception pour recevoir l'extrait biochimique résultant du module de mélange ou intermédiaire par vidage ou évacuation par gravité,
c.2) au moins un moyen turbine, comportant une pompe péristaltique permettant de recirculer l'extrait biochimique vers le module de pré-mélange ou supérieur et
d) au moins un moyen de surveillance et de traitement de données : vitesse d'agitation, dans chacun de tels au moins un premier, au moins un deuxième et au moins un troisième moyens d'agitation ; température, dans chacun de tels au moins un premier, au moins un deuxième et au moins un troisième moyens de réception et bioréacteur ; et pH dans chacun de tels au moins un premier, au moins un deuxième et au moins un troisième moyens de réception et au moins un bioréacteur, comprenant au moins un centre de données recevant à distance de telles données de vitesse d'agitation, de température et de pH de chacun de tels au moins un premier, au moins un deuxième et au moins un troisième moyens capteurs de vitesse d'agitation, moyens capteurs de température et moyens capteurs de pH de tels module de pré-mélange et module intermédiaire, un tel au moins un centre de données comprenant au moins une base de données, les données reçues étant stockées à partir de tels au moins un premier, au moins un deuxième et au moins un troisième moyens capteurs de vitesse d'agitation, capteurs de température et capteurs de pH, et au moins un processeur traitant de telles données et envoyant au moins un signal de sortie en réponse à de tels au moins un premier, au moins un deuxième et au moins un troisième moyens de contrôle de vitesse d'agitation, moyens de contrôle de température et moyens de contrôle de pH, après avoir comparé de telles données reçues avec des données de référence et prédéfinies stockées, déclenchant un actionnement manuel ou automatisé, dans un ou plusieurs de tels au moins un premier, au moins un deuxième et au moins un troisième moyens de contrôle de vitesse d'agitation, moyens de contrôle de pH ou moyens de contrôle de température lorsqu'une différence est confirmée entre de telles données reçues et de telles données de référence ou prédéfinies stockées.

2. Système selon la revendication 1, de tels au moins un premier et au moins un deuxième moyens de réception d'échantillon d'un tel module de pré-mélange ayant des vannes situées au niveau de la partie supérieure pour alimenter en/faire entrer de tels liquides S et G.

3. Système selon la revendication 1, un tel moyen de régulation de température à l'intérieur d'un tel bioréacteur comprenant un ventilateur et une résistance électrique.

4. Système selon la revendication 1, de tels premier et deuxième moyens de réception d'échantillon étant en verre.

5. Système selon la revendication 1 comprenant en outre un moyen émettant des signaux sonores ou auditifs ou les deux avertissant un utilisateur qu'il s'est écoulé un temps suffisant jusqu'à atteindre une homogénéité de 50 % à l'intérieur du module intermédiaire.

6. Système selon la revendication 1, un tel moyen émettant des signaux sonores ou auditifs ou les deux avertissant un utilisateur qu'il s'est écoulé un temps suffisant jusqu'à atteindre une homogénéité de 90 %.

7. Système selon la revendication 1, un tel signal sonore ou auditif ou les deux avertissant l'utilisateur que des niveaux thermiques prédéfinis ont été dépassés.

8. Système selon la revendication 1, un tel équipement de quantification comprenant un photomètre, un four à micro-ondes ménager, une pompe Parr et en outre un thermoréacteur.

9. Procédé de quantification à distance et en temps réel de la bioaccessibilité de métaux et de métalloïdes dans un échantillon de sol contaminé :
a) la préparation de liquides organiques et enzymatiques choisis parmi la salive (S), la bile (B), le liquide gastrique (G) et le liquide duodénal (D), selon les étapes suivantes :
a.1) la préparation d'un tel liquide organique S, l'ajout d'eau notamment de l'eau ultra pure, sous agitation jusqu'à atteindre une homogénéisation de 90 % et pour maintenir la température à 28 °C, en un rapport en volume/volume de 3/10, et l'ajout d'un agent oxydant pour réguler la valeur de pH dans une plage allant de 6,0 à 7,0, si nécessaire, puis le chauffage progressif du mélange jusqu'à atteindre 37 °C, un tel agent oxydant étant NaOH ;
a.2) la préparation d'un tel liquide organique G, l'ajout d'eau sous agitation jusqu'à atteindre une homogénéité de 90 % et pour maintenir la température à 28 °C, en un rapport en volume/volume de 37/80, et l'ajout d'un agent réducteur acide pour réguler la valeur de pH dans une plage allant de 1,0 à 1,2, si nécessaire, puis le chauffage progressif du mélange jusqu'à atteindre 37 °C ;
a.3) la préparation d'un tel liquide organique B, l'ajout d'eau notamment de l'eau ultra pure en agitant jusqu'à atteindre une homogénéité de 90 % et pour maintenir une température de 28 °C, en un rapport en volume/volume de 3/10, et l'ajout d'un agent réducteur acide pour réguler la valeur de pH dans une plage allant de 7,8 à 8,2, si nécessaire, puis le chauffage progressif du mélange jusqu'à atteindre 37 °C ;
a.4) la préparation d'un tel liquide organique D, l'ajout d'eau, de préférence de l'eau ultra pure sous agitation jusqu'à atteindre une homogénéité de 90 % et pour maintenir une température de 28 °C, en un rapport en volume/volume de 37/60, et l'ajout d'un agent réducteur acide pour réguler la valeur de pH dans une plage allant de 7,2 à 7,6, si nécessaire, puis le chauffage progressif du mélange jusqu'à atteindre 37 °C,
un tel agent de régulation acide de l'une quelconque des étapes a.2 à a.4) étant l'acide chlorhydrique ;
b) la préparation d'un échantillon de sol contaminé en le séchant à une température de 40 °C jusqu'à atteindre une humidité de 0 % et en le tamisant jusqu'à une taille inférieure à 250 micromètres,
c) le mélange d'un tel échantillon de sol résultant de l'étape b) avec un tel liquide résultant S de l'étape a.1) et un tel liquide résultant G résultant de l'étape a.2) et la réaction à l'intérieur du bioréacteur pendant un certain temps, puis le mélange résultant est ajouté pour réagir à l'intérieur du réacteur avec un tel liquide B résultant de l'étape a.3) et un tel liquide D résultant de l'étape a.4) pour maintenir la température d'un tel bioréacteur dans une plage allant de 37 à 39 °C, de préférence à 37 °C, et où de tels mélanges sont effectués sous agitation jusqu'à obtenir une homogénéité de plus de 50 %, de préférence de 90 %, par les sous-étapes suivantes :
c.1) le mélange d'un tel échantillon de sol contaminé préparé à l'étape b) renfermant du liquide S préparé à l'étape a.1) en un rapport en poids/volume d'un tel échantillon de sol sur un tel liquide S (mg/ml) allant de 100 à 15, sous agitation
c.2) l'ajout à un tel mélange résultant de l'étape c.1) de liquide G préparé à l'étape a.2), en un rapport en volume sur volume S:G de 2:3, sous agitation et régulation de pH à une valeur allant de 1,15 à 1,25, de préférence, à une valeur de pH de 1,2, et la réaction pendant au moins 1 heure,
c.3) l'ajout à un tel mélange résultant de l'étape c.2) de liquide D préparé à l'étape a.3), en un rapport en volume sur volume S:G:D de 2:3:6, sous agitation ;
c.4) l'ajout à un tel mélange résultant de l'étape c.3) de liquide B préparé à l'étape a.4), en un rapport en volume sur volume S:G:D:B de 2:3:6:2, sous agitation et régulation de la valeur de pH dans une plage allant de 5,8 à 6,8, de préférence,
à une valeur de pH de 6,3, et la réaction pendant au moins 4 heures,
d) la centrifugation des mélanges résultant de l'étape gastrique ou étape c.2) ou résultant de l'étape gastro-intestinale ou étape c.4), l'ajout d'un agent oxydant et la préparation de dilutions allant de 1:200 à 1:10 avec les mélanges et l'une de :
e) l'élimination de matière organique de telles dilutions de l'étape d) en soumettant la dilution à :
e.1) un milieu organique acide pour une digestion acide,
e.2) une digestion par désintégration en métal à :
e.2.1) 120 °C dans un thermoréacteur (méthode Crack Set de Merck) ou
e.2.2) par digestion acide par four à micro-ondes de laboratoire (type Milestone), une telle valeur de pH étant régulée dans une plage allant de 6 à 7 avant de quantifier l'un de Fe, Pb, Zn, Cu, Ni, Cd ou As, dans un photomètre,
f) la quantification par séparation d'un tel métal ou métalloïde choisi parmi (Cu), le fer (Fe), le zinc (Zn) et le plomb (Pb), le nickel (Ni), le cadmium (Cd) et l'arsenic (As), de tels échantillons résultant de l'étape e), dans :
f.1) un photomètre, un four à micro-ondes ménager et une pompe Parr,
f.2) un moyen de quantification mobile choisi parmi un canon à rayons X ou de fluorescence des rayons X ou
f.3) un moyen de quantification de métaux lourds choisi parmi la spectroscopie de masse à plasma (ICP) ou l'absorption atomique,
la valeur de pH étant régulée avant de quantifier un tel métal dans un photomètre à l'étape f.1).

10. Procédé selon la revendication 9, un tel sol contaminé étant choisi parmi du sol de rejet abandonné en ville.

11. Procédé selon la revendication 9, un tel sol contaminé étant choisi parmi du sol minier de rejet.

12. Procédé selon la revendication 9, les étapes a) et c) étant répétées autant de fois que cela est jugé nécessaire, les liquides S, B, D, G préparés étant mélangés avec le mélange résultant de l'étape c) avant chaque cycle se répétant.

13. Procédé selon la revendication 9 comprenant en outre l'émission de signaux visuels ou sonores ou les deux pour avertir un utilisateur qu'il s'est écoulé un temps suffisant jusqu'à atteindre une homogénéité du mélange de plus de 50 % soit lorsqu'un tel échantillon de sol est mélangé avec S et G soit lorsqu'un tel échantillon est mélangé avec S, G, B et D et un tel temps suffisant étant de 1 heure pour un tel mélange d'un tel échantillon de sol avec S et G.

14. Procédé selon la revendication 9, un tel signal visuel ou sonore ou les deux avertissant un utilisateur qu'il s'est écoulé un temps suffisant jusqu'à atteindre une homogénéité de 90 % et un tel temps suffisant étant de 4 heures pour un tel mélange d'un tel échantillon de sol avec S, G, B et D.

15. Procédé selon la revendication 9, une telle valeur de pH étant régulée avec NaOH et HCl avant la quantification de Fe.

16. Procédé selon la revendication 9, une telle valeur de pH étant régulée avec NH₃ dilué et HNOs dilué avant de quantifier Pb.

17. Procédé selon la revendication 9, une telle valeur de pH étant régulée avec NaOH et H₂SO₄ avant de quantifier l'un de Zn, Cu, Ni, Cd ou As.

18. Procédé selon la revendication 9, une telle digestion par désintégration de métal de l'étape e) étant mise en oeuvre dans un thermoréacteur avec des réactifs ajoutés en fonction du métal que l'on souhaite quantifier et selon les instructions du fabricant.

19. Procédé selon la revendication 9, une telle valeur de pH étant régulée dans une plage allant de 3 à 6 avant de quantifier Pb, à l'étape f.1).

20. Procédé selon la revendication 9, une telle valeur de pH étant régulée dans une plage allant de 1 à 10 pour quantifier Fe, à l'étape f.1).

21. Procédé selon la revendication 9, une telle valeur de pH étant régulée dans une plage allant de 4 à 10 pour quantifier Zn, à l'étape f.1).

22. Procédé selon la revendication 9, une telle valeur de pH étant régulée dans une plage allant de 3 à 10 pour quantifier Cu, à l'étape f.1).

23. Procédé selon la revendication 9, une telle valeur de pH étant régulée dans une plage allant de 3 à 8 avant de quantifier Ni, à l'étape f.1).
